# EUROPEAN PATENT APPLICATION

(11) **EP 2 327 412 A1**
(43) Date of publication of application: **01.06.2011**
(21) Application number: 09014837.0
(22) Date of filing: 30.11.2009
(51) Int. Cl.: A61K 38/17, A61P 25/00

(54) **Treatment of nerve injuries**

(71) Applicant: Universitätsklinikum Hamburg-Eppendorf, 20246 Hamburg (DE)
(72) Inventor: Schachner, Melitta, 20251 Hamburg (DE); Kleene, Ralf, 22529 Hamburg (DE); Mishra, Bibhudatta, 22529 Hamburg (DE)
(74) Representative: UEXKÜLL & STOLBERG

(57) **Abstract**

This invention relates to treatment of nerve injuries and regeneration of nerves and nerve function in the acutely and chronically lesioned central and peripheral nervous system. The invention provides a pharmaceutical composition comprising histone, e.g., H1. It was surprisingly shown that histones, in particular histone H1, can be used for the preparation of a pharmaceutical composition for treatment and/or prevention of acute and chronic injuries to the central and peripheral nervous system, for promoting growth and/or regrowth of nerves and enhancing formation of synapses and synaptic plasticity, and for neuroprotection.

SEQUENCE LISTING

<110> Universitätskrankenhaus Hamburg-Eppendorf

<120> Treatment of Nerve Injuries

<130> P81481

<160> 11

<170> PatentIn version 3.4

<210> 1
<211> 14
<212> PRT
<213> Artificial

<220>
<223> scFv linker region

<400> 1

Ser Ser Gly Gly Gly Gly Ser Gly Gly Gly Gly Ser Gly Gly
1               5                   10

<210> 2
<211> 5
<212> PRT
<213> Artificial

<220>
<223> scFv linker region

<400> 2

Ser Ser Gly Gly Gly
1               5

<210> 3
<211> 21
<212> DNA
<213> Artificial

<220>
<223> Primer

<400> 3
cagccggcca tggcccaggt g                                                21

<210> 4
<211> 45
<212> DNA
<213> Artificial

<220>
<223>  Primer

<400>  4
gagtcaccgt gcactgcctc caccactcga gacggtgacc agggt                    45


<210>  5
<211>  9
<212>  PRT
<213>  Artificial

<220>
<223>  scFv linker region

<400>  5

Leu Asn Asp Gly Pro Val Thr Ser Ala
1               5


<210>  6
<211>  6
<212>  PRT
<213>  Artificial

<220>
<223>  scFv linker region

<400>  6

Gly Leu Pro Ile Asp Ser
1               5


<210>  7
<211>  5
<212>  PRT
<213>  Artificial

<220>
<223>  scFv linker region

<400>  7

Ser Pro Phe Glu Ser
1               5


<210>  8
<211>  12
<212>  PRT
<213>  Artificial

<220>
<223>  peptide

<400>  8

Thr Ser Gly Pro Pro Val Ser Glu Leu Ile Thr Lys
1               5                   10

<210>  9
<211>  11
<212>  PRT
<213>  Artificial

<220>
<223>  peptide

<400>  9

Ala Leu Ala Ala Ala Gly Tyr Asp Val Glu Lys
1               5                   10


<210>  10
<211>  17
<212>  DNA
<213>  Artificial

<220>
<223>  Primer

<400>  10
caggaaacag ctatgac                                                    17


<210>  11
<211>  17
<212>  DNA
<213>  Artificial

<220>
<223>  Primer

<400>  11
gaattttctg tatgagg                                                    17

## Description

This invention relates to treatment of nerve injuries, in particular, traumatic, acute and/or chronic lesions of the central and peripheral nervous system, and to regeneration of nerves and nerve function. The invention provides a pharmaceutical composition comprising histone, in particular, histone H1. It was surprisingly shown that histones can be used for the preparation of a pharmaceutical composition for treatment and/or prevention of acute and chronic injuries to the central and peripheral nervous system, for promoting growth and/or regrowth of nerves and enhancing formation of synapses and synaptic plasticity, and for neuroprotection.

Polysialic acid (PSA) is known to play a crucial role in nervous system development and function. It has also been associated with regeneration of nerve function. PSA is a long, linear polymer composed entirely of negatively charged sialic acid ('5-N-acetylneuraminic acid) in α2,8 linkage, and belongs to a class of functionally important anionic glycans (Rutishauser et al., 2008). Because of its high negative charge, PSA is highly hydrated and has therefore been suggested to attenuate cellular interactions and increase motility (Mühlenhoff et al., 1998).

In the mammalian brain, PSA is predominantly or probably exclusively attached to the neural cell adhesion molecule NCAM. So far, only neuropilin-2 has been identified in human dendritic cells as another carrier of PSA in mammalia (Curreli et al., 2007), but it remains to be shown whether neuropilin-2 expressed in brain also carries PSA. PSA is associated with morphogenetic changes during developmental processes such as cell migration, synaptogenesis, axonal growth, and branching, whereas PSA persists in the adult brain only in structures that display a high degree of functional plasticity (Rutishauser et al., 2008; Kleene and Schachner, 2004). During embryonic development, PSA is expressed abundantly on Schwann cells and axons in the peripheral nervous system, while its expression decreases in adulthood. After nerve injury in adult rodents, PSA is upregulated in axons and skeletal muscle fibres (Olsen et al., 1995; Mehanna et al., 2009a). Additionally, *in vitro* and in vivo functional assays using PSA mimetics and/or the bacterial homolog of PSA, colominic acid, indicated that PSA is involved in myelination and regeneration of peripheral and central nerves (Jungnickel et al., 2009; Mehanna et al., 2009a and 2009b).

In the adulthood, PSA plays an important role in nervous system function and in synaptic plasticity. The functional and developmental defects observed upon ablation of PSA in mouse mutants lacking the main PSA carrier NCAM or the polysialyltransferases essential for the synthesis of PSA underscore the functional importance of PSA. In addition, the effects observed upon application of endoneuraminidase N which specifically degrades PSA further documented the functional plasticity of PSA. Although much is known about the functions of PSA which have been intensively studied, only a few possible binding partners have been described so far: BDNF (Muller et al., 2000), heparan sulfate proteoglycans (Storms and Rutishauser 1998), AMPA receptors (Vaithianathan et al., 2004), NR2B subunit containing NMDA receptors (Hammond et al., 2006) and estradiol (Garcia-Sequra et al., 1995). BDNF is a neurotrophic factor that shows a direct binding to PSA *in vitro* (Kanato et al., 2008) and promotes neuronal survival and differentiation during development (Sofroniew et al., 2001). Heterophilic binding of PSA-NCAM to the heparan sulfate proteoglycans has a positive effect on cell adhesion (Storms and Rutishauser 1998). The interaction between PSA and AMPA receptors results in age-dependent potentiation of AMPA receptor, which might modulate signalling via AMPA receptors in immature neurons and thereby affecting their development (Vaithianathan et al., 2004), while the interaction of PSA with NR2B subunit containing NMDA receptors prevents excitotoxicity (Hammond et al., 2006). The interaction of PSA with estradiol appears to participate in the estradiol-induced changes of glial cells with a polygonal shape to process-bearing cells (Garcia-Sequra et al., 1995).

It was recently shown that a PSA-mimicking peptide has positive effects on recovery after femoral nerve and spinal cord lesion and synaptic plasticity in mice (Mehanna et al., 2009a and 2009b; Marino et al., 2009; Papastefanaki et al., 2007; Senkov et al., 2006; Stoenica et al., 2006). This peptide improves myelination but does not influence motoneuron survival and regrowth. Brief low-frequency electrical stimulation of the injured femoral nerve also leads to long-lasting enhancement of axonal regrowth (Al-Majed et al., 2000; Brushart et al., 2005) and functional recovery (Ahlborn et al., 2007).

In light of the state of the art, the present inventors addressed the problem of providing alternative compounds that improve nerve regeneration or that might improve motoneuron regeneration and that may be used in therapy of nerve injury.

The present invention surprisingly identifies histone H1 as novel interaction partner of PSA and shows direct binding of histone H1 to PSA.

The invention therefore provides a pharmaceutical composition comprising a histone, in particular for treatment or prevention of nerve injury or for reducing the risk of nerve injury. The histone may be selected from the group consisting of histone H1, H2A, H2B, H3, and H4. Preferably, the histone is histone H1.

The pharmaceutical composition may comprise one or more different histones or a nucleosome, e.g., in the form of optionally digested chromatin. In the context of this invention "a" is not meant to be limiting to "one" unless explicitly specified, it can thus stand for one, two, three, four, five or more.

In one embodiment, the histone is a human histone, in particular, human histone H1, especially if humans are to be treated. However, alternatively, e.g., bovine histone, rat or murine histone or ape histone, such as chimpanzee histone may be used. There are eleven known mammalian subtypes of linker histone, H1.0, H1.1, H1.2, H1.3, H1.4, H1.5, H1.X, H1.t H1.t2 HILS and H1oo, as well as reptilian and amphibian H5, which are all referred to herein as H1. Preferably, H1 is selected from H1.1, H1.2, H1.3, H1.4 and H1.5, who all have an amino acid identity of at least 70% to H1.4. There is a high homology between species, e.g., human and murine histone H1 have 94% amino acid identity. Histone H1 preferably is human histone H1 or a variant or derivative thereof comprising a sequence with at least about 70% amino acid identity, at least about 80% amino acid identity, at least about 90% amino acid identity or at least about 95% or 99% amino acid identity to human H1.4. Preferably, the variant or derivative is capable of specifically binding to PSA and/or colominic acid, as tested in the present application e.g., by the ELISA with binding to colominic acid. The histone may be non-phosphorylated, phosphorylated or derivatized, e.g., PEGylated or biotinylated.

The histone or histones may be isolated from biological samples, e.g., from bovine thymus or cell culture, or produced recombinantly, e.g., in insect cells, COS cells, yeast or *E. coli.* Methods for recombinant production of histone H1 are disclosed, e.g., in US 2003/0078204.

Histone H1 was initially described as a classical nuclear protein that is involved as a linker histone in the organization of nucleosomes which mediate DNA-packing (Doenecke et al., 1997), but it also has been discovered in extranuclear (Parseghian and Luhrs, 2006) and even extracellular locations. Previously it has been reported that immunohistochemistry revealed a staining of histone H1 at neuronal cell bodies of cultured primary mouse cortical neurons, suggesting that histone H1 was located at the cell surface of neurons (Bolton and Perry, 1997). However, an extracellular matrix localization of histone H1 had not been shown for neuronal cells.

Extracellular histones, among them histone H1, have previously been detected at the surface of human monocytes (Holers and Kotzin, 1985), activated human peripheral blood lymphocytes (Watson et al., 1995) and cultured T-cells (Watson et al., 1995; Watson et al., 1999) as well as a macrophage cell line where it interacts with thyroglobulin and may mediates thyroglobulin endocytosis (Brix et *al.,* 1998). The trafficking of histone proteins from the nucleus to the cytoplasm, cell surface or extracellular environment is not known yet, but since penetration of extracellular histone through the plasma membrane and into the cell has been demonstrated (Hariton-Gazal et al., 2003) it may be possible that a similar pathway may exist for the export of histones.

Extracellular histones including H1 have been shown to bind to cell surface heparan sulfate proteoglycans (Watson et al., 1999) and lipopolysaccharide (LPS) (Hampton et al., 1988; Bolton and Perry, 1997) which upregulate the expression of histones on the cell surface of monocytes (Emlen et al., 1992). In addition, extracellular histones, in particular histone H1, have also been reported to exhibit antibacterial activity (Hirsch, 1958; Hiemstra et al., 1993) which is antagonized by acid polysaccharides like heparin. There are also indications that histone H1 binds to amyloid precursor protein (Potempska et al., 1993) and amyloid-like motifs, such as β-amyloid and α-synuclein (Duce et al., 2006) and it was found associated with amyloid plaques (Duce et al., 2006). It has also been discussed that it binds to the pathogenic scrapie form of the prion protein and it has been shown that histone H1 is upregulated in brains of mice showing clinical sign of scrapie and an increased immunostaining of histone H1 was observed in region of pathology (Bolton et al., 1999). It is noteworthy to mention that prion protein as well as amyloid precursor protein binds to heparan sulfate proteoglycans which play a role in the pathogenesis of Alzheimer's disease, Scrapie or related diseases (van Horssen et al., 2003; Horonchick et al., 2005), and which bind to PSA-NCAM.

Histone H1 has been localized on the plasma membrane or in the extracellular matrix of skeletal muscle cells, and the level of extracellular histone H1 increases during muscle differentiation (Henriquez et al., 2002). The extracellular pool of histone H1 colocalizes with heparan sulfate proteoglycan perlecan in the extracellular matrix of myotube cultures and in regenerating skeletal muscle. In addition, histone H1 was identified as endogenous extracellular ligand for muscle cell heparan sulfate proteoglycans which regulate skeletal muscle differentiation. Extracellular histone H1 strongly stimulated myoblast proliferation via a heparan-sulfate-dependent mechanism, suggesting that extracellular histone H1 in conjunction with heparan sulfate proteoglycans plays a functional role during skeletal muscle development and regeneration. The role of extracellular histone H1 in regulating cell proliferation and differentiation is also supported by its anti-proliferative effects on cancer cells (Vani et al., 2006). Interestingly, it has effects on estrogen receptor status of human breast cancer cells (Vani and Devi, 2005); suggesting that, together with PSA, which is a putative binding partner of estradiol, it regulates estrogen-induced effects on proliferation.

It has previously been suggested to use Histone H1 as an antimicrobial agent (e.g., US 6,884,423) or for treatment of rheumatoid arthritis (US 6,204,242).

The present inventors showed that histone H1 is additionally present extracellularly at the cell surface of cerebellar neurons and Schwann cells, and that histone H1 has a beneficial effect on neural precursor cell migration, neurite outgrowth as well as Schwann cell proliferation and process elongation; suggesting that extracellular histone H1 plays an important role during development and regenerating of the peripheral and central nervous system and in addition enhances synaptic plasticity. The better functional recovery and preferential motoneuron reinnervation after peripheral nerve injury by histone H1 indicates that it has a functional role in regeneration *in vivo* and can be used therapeutically.

In one aspect, the pharmaceutical composition of the present invention comprising histone, in particular, histone H1, is for treatment or prevention of nerve injury, such as acute and chronic trauma, central and peripheral nervous system regeneration and neurodegenerative diseases. The pharmaceutical composition may also be for enhancing learning and memory.

The invention also provides the use of a histone for the preparation of the pharmaceutical composition comprising a histone, e.g., histone H1, for the treatment and/or prevention of nerve injury.

The invention also provides a method for treating and/or preventing nerve injury, wherein a pharmaceutical composition comprising an effective amount of an isolated histone, e.g., histone H1, is administered to a patient in need thereof.

The histone can be in the form of a protein, alternatively, a gene transfer vector for expression of a histone and/or a histone expressing cell may be used in the pharmaceutical composition of the invention.

The nerve injury may be injury in the peripheral and/or central nervous system. The nerve injury may comprise a lesion to the brain, the spinal cord, sensory neurons and/or motoneurons. The nerve injury may be inflicted by an operation, a disease, in particular a degenerative disease and/or trauma.

The composition may be formulated for administration, e.g., by injection or intraoperational administration, preferably locally, e.g., at the site of the lesion. If the injury is inflicted by an operation, or if there is a danger that an operation may inflict an injury to a nerve, the composition is preferably administered intraoperationally, e.g., similar to the administration in the example.

It can e.g. be beneficial to administer the pharmaceutical composition of the invention in the context of prostate surgery/prostatectomy to reduce complications due to nerve injury, in the context of surgery near the spinal cord or brain surgery, e.g., due to a brain tumor. When injury due to a trauma is treated by an operation, this also opens the option of intraoperational administration. Other options are infusion into the lumbar spinal cord or into the brain, e.g. by using an Alzet osmotic pump. Alternatively, the pharmaceutical composition of the invention may be administered by injection, e.g., at the site of the lesion or by intravenous injection, or by infusion into the diseased tissue.

In the context of the invention, it is also possible to administer to the patient cells, preferably autologous or syngeneic or histologically compatible cells, e.g., fibroblasts or stem cells, which are genetically engineered for expressing histones, preferably overexpressing histones, e.g., histone H1 and/or the histones described above, or to administer a gene transfer vector e.g. an adenoviral vector or adeno-associated vector engineered to induce expression of said histones. Preferably, in the context of gene therapy of the invention, an extracellular expression of histones is achieved. The skilled person is aware of suitable vectors, promoters, signal sequences for extracellular expression and methods. If appropriate, a promotor suitable for expression in cells of the nervous system may be used.

Similar positive effects on recovery after femoral nerve lesion in mice as with histone H1 were observed for PSA using PSA mimicking peptides (Mehanna et al., 2009a). The time course of improvement is similar for histone H1, with positive effects becoming apparent later, i.e. at 2-3 months after injury and treatment, leading to the question how, e.g., a single intraoperative application of a protein can produce this late-appearing and long-lasting effect of histone H1. Local availability of a protein is most likely limited to hours or at best days after application due to its degradation. Therefore, without intending to be bound by the hypothesis, it is proposed that histone H1 modulates early cellular and molecular responses to injury such that subsequent regeneration processes are positively influenced over weeks. Based on the *in vitro* data of enhanced proliferation and process elongation of Schwann cells shown in the Examples, it can be concluded that histone H1 stimulates an early response of these cells to injury in vivo resulting in priming of injured femoral nerve at the lesion site to regenerate.

The pharmaceutical composition of the present invention may thus be administered to the patient once, preferably as soon as possible after the lesion has occurred or has been detected. It may e.g., be administered directly (i.e. within 10 min or within 1 or 2 hours) after this time point, or within 1, 2, 3, 4 or 5 days after this time point. Alternatively, the pharmaceutical composition of the present invention may be administrated constantly over a time period of days or weeks, e.g., using an Alzet osmotic pump, infusion or grafting of histone-expressing cells, e.g stem cells or fibroblasts that are transfected with histone-expressing cell or engrafting histone encoding adeno-associated viral (AAV) vector. Such transfected cells may be allogeneic or syngeneic cells.

The pharmaceutical composition of the invention may also be administered before a lesion is formed or detected, to reduce the risk of nerve injury. Prevention of nerve injury can thus be a reduction of a risk of nerve injury. The invention in one aspect provides a method of reducing the risk of nerve injury, e.g., due to an operation, wherein the composition of the invention is administered at the site of the risk of nerve injury in the context of an operation, e.g., prostate surgery or brain surgery.

The pharmaceutical composition of the invention may also be administered twice, three or four times or more. It may also be formulated as a sustained release formulation. Of course, if there is more than one lesion, the composition may be administered at more than one lesion.

Since neurite outgrowth was promoted by histone H1, it is likely that regeneration of the injured nerve by histone H1 is accompanied by an enhanced axon regrowth *in vivo.* This notion was confirmed by retrograde-labeling of motoneurons showing that survival of motoneurons, axon regrowth and precision of reinnervation was significantly enhanced.

The diverse and, importantly, long-lasting effects of the histone H1 treatment suggest its involvement in basic signaling mechanisms which are either PSA-dependent or PSA-independent. PSA associated with NCAM acts as a positive modulator of NCAM functions by inhibiting the cis-interactions of NCAM and making more NCAM available for trans-interactions for instance with heparan sulfate proteoglycans which promote axonal growth and synaptic plasticity. On the other hand, histone H1 may bind to PSA-NCAM and trigger its diverse functions via NCAM-mediated signal transduction and/or it may bind to other binding partners such as heparan sulfate proteoglycans and trigger PSA- and NCAM-independent signal transduction pathways. Interaction of histone H1 with NCAM binding partners, such as heparan sulfate proteoglycans and prion protein, may modulate the interaction with PSA and its binding partners such as BDNF and further influence PSA-dependent signaling transduction pathways. The diverse functions of histone H1 in different neural cell types and its interaction with different binding partners show that it plays a central role in modulating and integrating various cellular functions associated with cell survival, proliferation and regeneration in a PSA-dependent and -independent manner.

The pharmaceutical composition of the present invention thus has been shown to be useful for inducing neurite outgrowth, for promoting process formation of Schwann cells, for promoting proliferation of Schwann cells, for enhancing migration and differentiation of neural progenitor cells (neural stem cells), for promoting survival of neurons, for promoting stem cell functions and/or promoting functional recovery of imperiled and/or injured neurons in acute and chronic injuries (including neurodegeneration) as well as enhancing synapse formation and synaptic plasticity. Histones can thus also be used for enhancing learning and/or memory, e.g., in treatment of diseases associated with deficiencies in learning and/or memory.

The following examples are intended to illustrate the present invention but not to limit the scope thereof. All publications cited herein are herewith incorporated herein by reference.

### REFERENCES

Ahlborn P, Schachner M, Irintchev A (2007) One hour electrical stimulation accelerates functional recovery after femoral nerve repair. Exp Neurol 208:137-144.
Al-Majed AA, Neumann CM, Brushart TM, Gordon T (2000) Brief electrical stimulation promotes the speed and accuracy of motor axonal regeneration. J Neurosci 20:2602-2608.
Angata K, Huckaby V, Ranscht B, Terskikh A, Marth JD, Fukuda M (2007) Polysialic acid-directed migration and differentiation of neural precursors are essential for mouse brain development. Mol Cell Biol 27:6659-6668.
Asahara T, Lin M, Kumazawa Y, Takeo K, Akamine T, Nishimura Y (1999) Long-term observation on the changes of somatotopy in the facial nucleus after nerve suture in the cat: morphological studies using retrograde labeling. Brain Res Bull 49: 195-202.
Bolton SJ, Russelakis-Carneiro M, Betmouni S, Perry VH (1999) Non-nuclear histone H1 is upregulated in neurones and astrocytes in prion and Alzheimer's diseases but not in acute neurodegeneration. Neuropathol Appl Neurobiol 25:425-432.
Bolton SJ, Perry VH (1997) Histone H1; A neuronal protein that binds bacterial lipopolysaccharide. J Neurocytol 26:823-831.
Brix K, Summa W, Lottspeich F, Herzog V (1998) Extracellularly occurring histone H1 mediates the binding of thyroglobulin to the cell surface of mouse macrophages. J Clin Invest 102:283-293.
Brushart TM (1988) Preferential reinnervation of motor nerves by regenerating motor axons. J Neurosci 8:1026-1031.
Brushart TM, Jari R, Verge V, Rohde C, Gordon T (2005) Electrical stimulation restores the specificity of sensory axon regeneration. Exp Neurol 194:221-229.
Chazal G, Durbec P, Jankovski A, Rougon G, Cremer H (2000) Consequences of neural cell adhesion molecule deficiency on cell migration in the rostral migratory stream of the mouse. J Neurosci 20:1446-1457.
Cohen RS, Blomberg F, Berzins K, Siekevitz P (1977) The structure of postsynaptic densities isolated from dog cerebral cortex. J Cell Biol 74:181-203.
Curreli S, Arany Z, Gerardy-Schahn R, Mann D, Stamatos NM (2007) Polysialylated neuropilin-2 is expressed on the surface of human dendritic cells and modulates dendritic cell-T lymphocyte interactions. J Biol Chem 282:30346-30356.
Decker L, Avellana-Adalid V, Nait-Oumesmar B, Durbec P, Baron-Van Evercooren, A (2000) Oligodendrocyte precursor migration and differentiation: combined effects of PSA residues, growth factors, and substrates. Mol Cell Neurosci 16:422-439.
de la Cruz RR, Pastor AM, Delgado-Garcia JM (1994) Effects of target depletion on adult mammalian central neurons: morphological correlates. Neuroscience 58:59-79.
Doenecke D, Albig W, Bode C, Drabent B, Franke K, Gavenis K, Witt O (1997) Histones: genetic diversity and tissue-specific gene expression. Histochem Cell Biol 107:1-10.
Duce JA, Smith DP, Blake RE, Crouch PJ, Li QX, Masters CL, Trounce IA (2006) Linker histone H1 binds to disease associated amyloid-like fibrils. J Mol Biol 361:493-505.
Emlen W, Holers VM, Arend WP, Kotzin B (1992) Regulation of nuclear antigen expression on the cell surface of human monocytes. J Immunol 148:3042-3048.
Evans SV, Sigurskjold BW, Jennings HJ, Brisson JR, To R, Tse WC, Altman E, Frosch M, Weisgerber C, Kratzin HD (1995) Evidence for the extended helical nature of polysaccharide epitopes. The 2.8 A0 resolution structure and thermodynamics of ligand binding of an antigen binding fragment specific for 8)-polysialic acid. Biochemistry 34:6737-6744.
Franz CK, Rutishauser U, Rafuse VF (2005) Polysialylated neural cell adhesion molecule is necessary for selective targeting of regenerating motor neurons. J Neurosci 25: 2081-2091.
Frosch M, Görgen I, Boulnois GJ, Timmis KN, Bitter-Suermann D (1985) NZB mouse system for production of monoclonal antibodies to weak bacterial antigens: isolation of an IgG antibody to the polysaccharide capsules of Escherichia coli K1 and group B meningococci. Proc Natl Acad Sci USA 82:1194-1198.
Garcia-Segura LM, Cañas B, Parducz A, Rougon G, Theodosis D, Naftolin F, Torres-Aleman I (1995) Estradiol promotion of changes in the morphology of astroglia growing in culture depends on the expression of polysialic acid of neural membranes. Glia 13:209-216.
Griffiths AD, Williams SC, Hartley O, Tomlinson IM, Waterhouse P, Crosby WL, Kontermann RE, Jones PT, Low NM, Allison TJ (1994) Isolation of high affinity human antibodies directly from large synthetic repertoires. EMBO J 13:3245-3260.
Hammond MS, Sims C, Parameshwaran K, Suppiramaniam V, Schachner M, Dityatev A (2006) Neural cell adhesion molecule-associated polysialic acid inhibits NR2B-containing N-methyl-D-aspartate receptors and prevents glutamate-induced cell death. J Biol Chem 281:34859-34869.
Hampton RY, Golenbock DT, Raetz CR (1988) Lipid A binding sites in membranes of macrophage tumor cells. J Biol Chem 263:14802-14807.
Hariton-Gazal E, Rosenbluh J, Graessmann A, Gilon C, Loyter A (2003) Direct translocation of histone molecules across cell membranes. J Cell Sci 116:4577-4586.
Henriquez JP, Casar JC, Fuentealba L, Carey DJ, Brandan E (2002) Extracellular matrix histone H1 binds to perlecan, is present in regenerating skeletal muscle and stimulates myoblast proliferation. J Cell Sci 115:2041-2051.
Hiemstra PS, Eisenhauer PB, Harwig SS, van den Barselaar MT, van Furth R, Lehrer RI (1993) Antimicrobial proteins of murine macrophages. Infect Immun 61:3038-3046.
Hirsch JG (1958) Bactericidal action of histone. J Exp Med 108:925-944.
Holers VM, Kotzin BL (1985) Human peripheral blood monocytes display surface antigens recognized by monoclonal antinuclear antibodies. J Clin Invest 76:991-998.
Holliger P, Prospero T, Winter G (1993) "Diabodies": small bivalent and bispecific antibody fragments. Proc Natl Acad Sci USA 90:6444-6448.
Horonchik L, Tzaban S, Ben-Zaken O, Yedidia Y, Rouvinski A, Papy-Garcia D, Barritault D, Vlodavsky I, Taraboulos A (2005) Heparan sulfate is a cellular receptor for purified infectious prions. J Biol Chem 280:17062-17067.
Hu H (2000) Polysialic acid regulates chain formation by migrating olfactory interneuron precursors. J Neurosci Res 61: 480-492.
Hu H, Tomasiewicz H, Magnuson T, Rutishauser U (1996) The role of polysialic acid in migration of olfactory bulb interneuron precursors in the subventricular zone. Neuron 16:735-743.
Irintchev A, Simova O, Eberhardt KA, Morellini F, Schachner M (2005) Impacts of lesion severity and tyrosine kinase receptor B deficiency on functional outcome of femoral nerve injury assessed by a novel single-frame motion analysis in mice. Eur J Neurosci 22:802-808.
Jungnickel J, Brämer C, Bronzlik P, Lipokatic-Takacs E, Weinhold B, Gerardy-Schahn R, Grothe C (2009) Level and localization of polysialic acid is critical for early peripheral nerve regeneration. Mol Cell Neurosci 40: 374-381.
Kanato Y, Kitajima K, Sato C (2008) Direct binding of polysialic acid to a brain-derived neurotrophic factor depends on the degree of polymerization. Glycobiology 18:1044-1053.
Kleene R and Schachner M (2004) Glycans and neural cell interactions. Nat Rev Neurosci 5:195-208.
Kruse J, Mailhammer R, Wernecke H, Faissner A, Sommer I, Gorides C, Schachner M (1984) Neural cell adhesion molecules and myelin-associated glycoprotein share a common carbohydrate moiety recognized by monoclonal antibodies L2 and HNK-1. Nature 311:153-155.
Marino P, Norreel JC, Schachner M, Rougon G, Amoureux MC (2009) A polysialic acid mimetic peptide promotes functional recovery in a mouse model of spinal cord injury. Exp Neurol 219:163-174.
Mehanna A, Mishra B, Kurschat N, Schulze C, Bian S, Loers G, Irintchev A, Schachner M (2009a) Polysialic acid glycomimetics promote myelination and functional recovery after peripheral nerve injury in mice. Brain 132:1449-1462.
Mehanna A, Jakovcevski I, Acar A, Xiao M, Loers G, Rougon G, Irintchev A, Schachner M (2009b) Polysialic Acid Glycomimetic Promotes Functional Recovery and Plasticity After Spinal Cord Injury in Mice. Mol Ther, Oct 13. [Epub ahead of print]
Mühlenhoff M, Eckhardt M, Gerardy-Schahn R (1998) Polysialic acid: three-dimensional structure, biosynthesis and function. Curr Opin Struct Biol 8:558-564.
Muller D, Djebbara-Hannas Z, Jourdain P, Vutskits L, Durbec P, Rougon G, Kiss JZ (2000) Brain-derived neurotrophic factor restores long-term potentiation in polysialic acid-neural cell adhesion molecule-deficient hippocampus. Proc Natl Acad Sci USA 97:4315-4320.
Olsen M, Zuber C, Roth J, Linnemann D, Bock E (1995) The ability to re-express polysialylated NCAM in soleus muscle after denervation is reduced in aged rats compared to young adult rats. Int J Dev Neurosci 13:97-104.
Parseghian MH, Luhrs KA (2006) Beyond the walls of the nucleus: the role of histones in cellular signaling and innate immunity. Biochem Cell Biol 84: 589-604.
Papastefanaki F, Chen J, Lavdas AA, Thomaidou D, Schachner M, Matsas R (2007) Grafts of Schwann cells engineered to express PSA-NCAM promote functional recovery after spinal cord injury. Brain 130:2159-2174.
Patrzykat A, Zhang L, Mendoza V, Iwama GK, Hancock RE (2001) Synergy of histone-derived peptides of coho salmon with lysozyme and flounder pleurocidin. Antimicrob Agents Chemother 45:1337-1342.
Potempska A, Ramakrishna N, Wisniewski HM, Miller DL (1993) Interaction between the beta-amyloid peptide precursor and histones. Arch Biochem Biophys 304:448-453.
Rutishauser U (2008) Polysialic acid in the plasticity of the developing and adult vertebrate nervous system. Nat Rev Neurosci 9:26-35.
Senkov O, Sun M, Weinhold B, Gerardy-Schahn R, Schachner M, Dityatev A (2006) Polysialylated neural cell adhesion molecule is involved in induction of long-term potentiation and memory acquisition and consolidation in a fear-conditioning paradigm. J Neurosci. 26:10888-10898.
Simova O, Irintchev A, Mehanna A, Liu J, Dihné M, Bächle D, Sewald N, Loers G, Schachner M (2006) Carbohydrate mimics promote functional recovery after peripheral nerve repair. Ann Neurol 60:430-437.
Sofroniew MV, Howe CL, Mobley WC (2001) Nerve growth factor signaling, neuroprotection, and neural repair. Annu Rev Neurosci 24:1217-1281.
Stoenica L, Senkov O, Gerardy-Schahn R, Weinhold B, Schachner M, Dityatev A (2006) In vivo synaptic plasticity in the dentate gyrus of mice deficient in the neural cell adhesion molecule NCAM or its polysialic acid. Eur J Neurosci 23:2255-2264.
Storms SD, Rutishauser U (1998) A role for polysialic acid in neural cell adhesion molecule heterophilic binding to proteoglycans. J Biol Chem 273:27124-27129.
Vaithianathan T, Matthias K, Bahr B, Schachner M, Suppiramaniam V, Dityatev A, Steinhäuser C (2004) Neural cell adhesion molecule-associated polysialic acid potentiates AMPA receptor currents. J Biol Chem 279:47975-47984.
van Horssen J, Wesseling P, van den Heuvel LP, de Waal RM, Verbeek MM (2003) Heparan sulphate proteoglycans in Alzheimer's disease and amyloid-related disorders. Lancet Neurol 2:482-492. Vani G, Devi CS (2005) Effect of histone H1 on estrogen receptor status of human breast cancer MCF 7 cells. Mol Cell Biochem 272:151-155.
Vani G, Vanisree AJ, Shyamaladevi CS (2006) Histone H1 inhibits the proliferation of MCF 7 and MDA MB 231 human breast cancer cells. Cell Biol Int 30:326-331.
Waters HJ, Barnett G, O'Hanlon GM, Lowrie MB (1998) Motoneuron survival after neonatal peroneal nerve injury in the rat-evidence for the sparing effect of reciprocal inhibition. Exp Neurol 152:95-100.
Watson K, Edwards RJ, Shaunak S, Parmelee DC, Sarraf C, Gooderham NJ, Davies DS (1995) Extra-nuclear location of histones in activated human peripheral blood lymphocytes and cultured T-cells. Biochem Pharmacol 50:299-309.
Watson K, Gooderham NJ, Davies DS, Edwards RJ (1999) Nucleosomes bind to cell surface proteoglycans. J Biol Chem 274 (31):21707-21713.
US 6,204,242
US 6,884,423
US 2003/0078204

### LEGENDS TO THE FIGURES

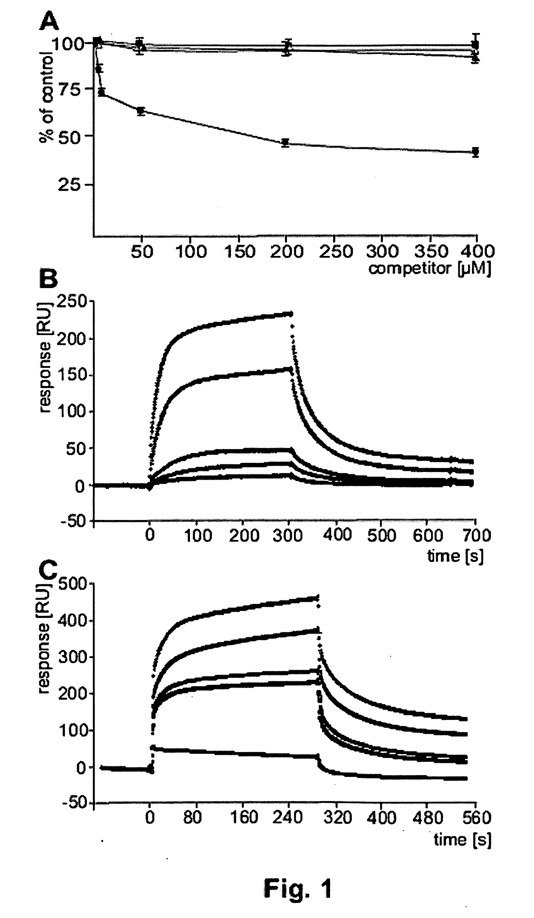

**Fig. 1****:** *Specificity of anti-idiotypic scFv antibodies directed against a PSA antibody.* **(A)** Substrate-coated PSA antibody 735 was pre-incubated with different concentrations of colominic acid (closed cycles), chondroitin sulfate A (closed triangles) or C (open triangles) or heparin (closed rectangles) and incubated with purified scFv antibody deriving from a phage with the sequence GLPIDS (SEQ ID NO:6) in the CDR3 region of the heavy chain which was isolated by screening a phage library of scFv antibodies using the PSA antibody 735. Binding of the scFv antibody to the PSA antibody was determined by ELISA and binding in the presence of competitor was related to the binding in the absence of competitor which was set to 100%. Mean values ± SD from three independent experiments are shown. **(B, C)** Surface plasmon resonance experiments were performed using immobilized PSA antibody 735 and different concentrations of scFv antibody for injection **(B)** or a constant amount of scFv antibody and different concentrations of colominic acid for injection **(C)**. Binding of scFv was monitored by measuring the response signal in arbitrary units (RU). A representative experiment is shown.

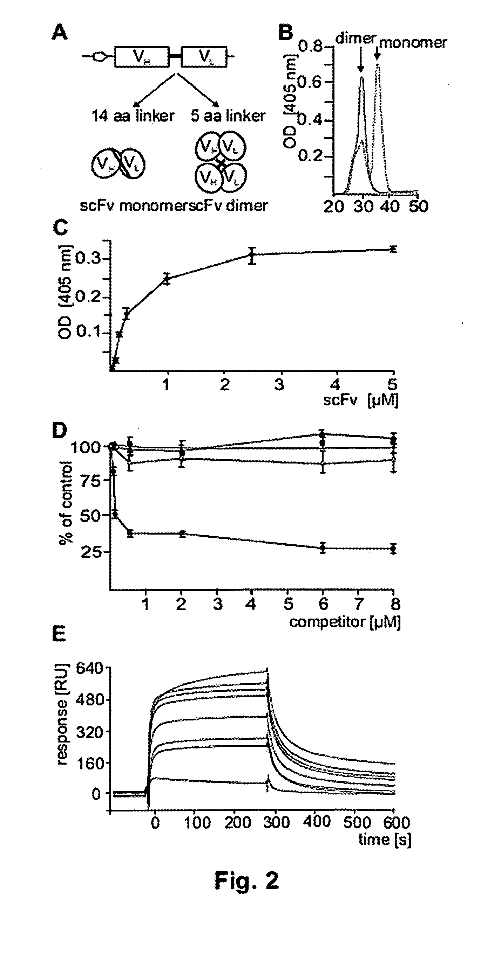

**Fig. 2****:** *Specificity of the PSA-mimicking scFv antibodies after linker truncation.* **(A)** The scFv antibody contains a linker of 14 amino acid (14 aa linker) between the V_{H} and V_{L} chains before truncation and 5 amino acids (5 aa linker) after truncation. The truncation should result in increased formation of scFv antibody dimers. **(B)** Purified scFv antibody without truncation having a linker region of 14 amino acids (dashed line) or the scFv antibody after truncation containing 5 amino acids (solid line) were applied to a Superdex-75 column and separated by FPLC. The retention times of the monomeric and dimeric forms are indicated. **(C)** ELISA with substrate-coated PSA antibody 735 using increasing concentrations of scFv antibody with linker truncation. **(D)** In competition ELISA, different concentrations of colominic acid (closed cycles), chondroitin sulfate A (closed triangles) or C (open triangles), and heparin (closed rectangles) and a constant amount of scFv antibody with linker truncation were incubated with substrate-coated PSA antibody 735. (C, **D)** Mean values ± SD from three independent experiments are shown for the binding of scFv antibody to the PSA antibody relative to the binding in the absence of competitor which was set to 100%. **(E)** Surface plasmon resonance experiments were performed using immobilized PSA antibody 735 and different concentrations of colominic acid together with a constant amount of scFv antibody with linker truncation for injection. Binding of scFv antibody in a representative experiment is shown. **(B-E)** Results obtained for an scFv antibody deriving from a phage with the sequence GLPIDS (SEQ ID NO:6) in the CDR3 region of the heavy chain are shown.

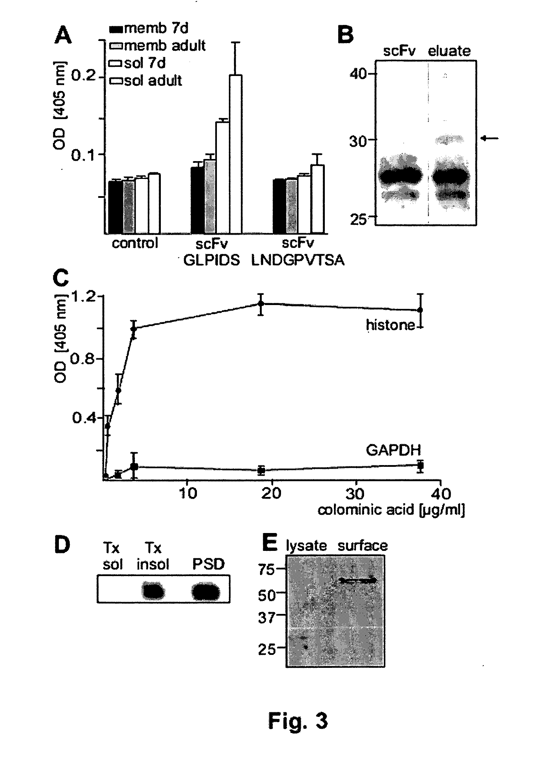

**Fig. 3****:** *Identification of histone H1 as extracellular membrane-attached PSA binding partner using a PSA-mimicking scFv antibody after linker truncation.* **(A)** Detergent-solubilized membrane (memb) and soluble (sol) brain proteins isolated from 7-day-old (7d) or 2-month-old (adult) mice were used as substrate-coat and incubated without (control) with scFv antibody with linker truncation deriving from phages containing either the sequence GLPIDS (SEQ ID NO:6) or LNDGPVTSA (SEQ ID NO:5) in the CDR3 region of the heavy chain. Mean values ± SD from three independent experiments are shown for the binding of scFv to soluble or membrane proteins. **(B)** Purified scFv antibody carrying the sequence GLPIDS (SEQ ID NO:6) was immobilized and used for affinity chromatography with membrane-attached brain proteins obtained by alkaline treatment of membranes. Proteins binding to the scFv antibody were eluted and subjected to silver staining together with purified scFv antibody as a control. The position of the 30 kDa protein band which was analyzed by mass spectrometry and identified as histone H1 is indicated by the arrow. **(C)** Histone H1 and GAPDH as negative control were substrate-coated and incubated with increasing concentrations of colominic acid. The binding of colominic acid was detected by ELISA using PSA antibody 735 and corresponding HRP-conjugated secondary antibody. Mean values ± SD from of three independent experiments carried out in triplicates are shown. **(D)** Triton X-100-soluble (Tx sol), Triton X-100-insoluble membrane proteins (Tx insol) and postsynaptic densities (PSD) were subjected to Western blot analysis using histone H1 specific polyclonal rabbit antibody. **(E)** Cultured live cerebellar neurons were incubated with biotinylation reagent. Biotinylated surface (surf) proteins were precipitated by streptavidin beads and subjected to Western blot analysis using a rabbit polyclonal histone H1 antibody. Total cell lysate (lys) was used as input control. The biotinylated histone H1 shows an apparent molecular weight of ∼60 kDa and is indicated by an arrow, while it is detectable as a ∼30 kDa protein in the cell lysate. This result suggests that extracellular histone H1 exists in a stable dimeric form.

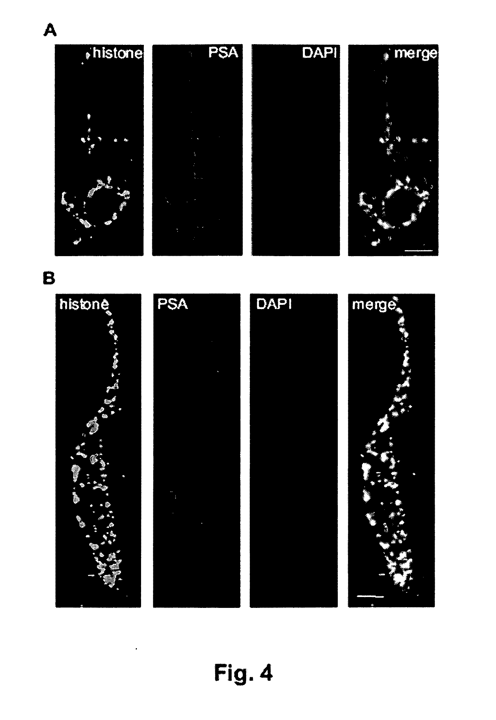

**Fig. 4****:** *Co-localization of histone H1 and PSA at the surface of live cerebellar neurons and Schwann cells.* **(A)** Cultured live cerebellar neurons **(A)** or Schwann cells **(B)** were immunostained with rabbit polyclonal histone H1 antibody, mouse monoclonal PSA antibody 735 and the corresponding fluorescent dye labeled secondary antibodies. After fixation and permeabilization of cells, nuclei were stained with DAPI. Superimpositions of histone H1 and PSA staining show partial co-localization (seen in yellow) at the surface of cell bodies and processes. Bars represent 10 µm.

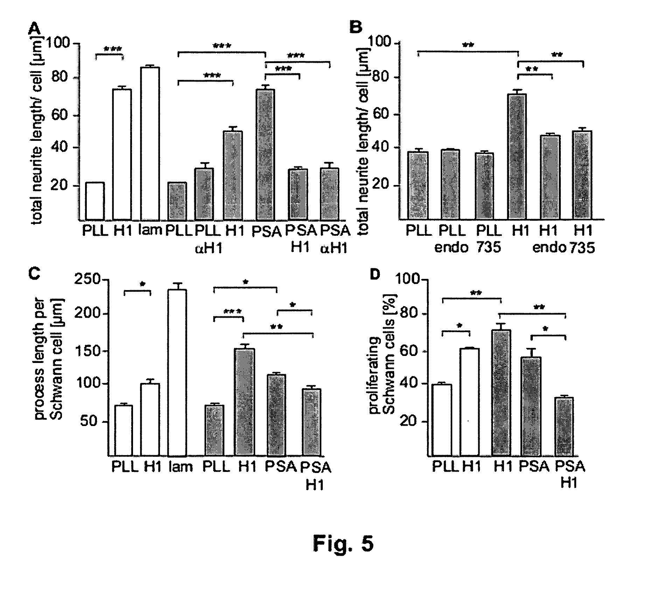

**Fig. 5****:** *Histone H1 affects neurite outgrowth of cerebellar neurons and process elongation and proliferation of Schwann cells in a PSA-dependent manner.* **(A)** Primary cultures of cerebellar neurons **(A, B)** or Schwann cells **(C, D)** were grown on substrate-coated PLL, histone H1 (H1) or laminin (lam) or on substrate-coated PLL in the presence of soluble PSA and/or histone H1 **(A-D)**, histone H1 antibody (αH1) **(A)**, PSA-specific antibody (735) or endoglycosidase N (endo) **(B)**. The total length of neurites per cerebellar neuron **(A, B)**, the process length per Schwann cell **(C)** and the number of BrdU labelled proliferating Schwann cells **(D)** were determined. **(A-D)** Asterisks, double asterisks and triple asterisks denote p<0.05, p<0.01 and p<0.001 obtained by two-tailed t test, respectively, from n= ∼100 neurons **(A, B)**, n= ∼100 **(C)** or n= ∼1000 **(D)** Schwann cells in three independent experiments.

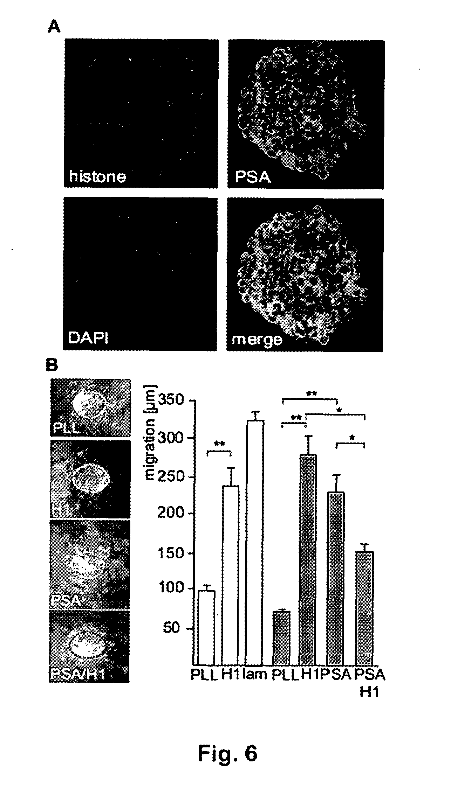

**Fig. 6****:** *Histone H1 and PSA are expressed by neurospheres and affect migration of neural precursor cells.* **(A)** Slices of neurospheres were stained with histone H1 and PSA specific antibodies followed by DAPI staining. Superimpositions of histone H1 and PSA staining show partial co-localization (seen in yellow) at the surface of neural precursor cells. Bar represents 10 µm. **(B)** Neurospheres were seeded onto substrate-coated PLL (PLL), histone H1 (H1) or laminin (lam) or on substrate-coated PLL in the presence of soluble PSA and/or histone H1. Micrographs displaying neurospheres (borders are indicated by dashed lines) with cells migrating out of the neurospheres in the absence (PLL) or presence of soluble PSA (PSA), soluble histone H1 (H1) or both soluble PSA and soluble histone H1 (PSA/H1) (left panel) and the quantification of the total distance reached by all migrating cells measured from the border of the neurosphere (right panel) are shown. Mean values + SD from three independent experiments are shown. Asterisks and double asterisks denotes p< 0.05, and p<0.01 respectively, obtained by the two-tailed t test from 20 neurospheres per group from three independent experiments.

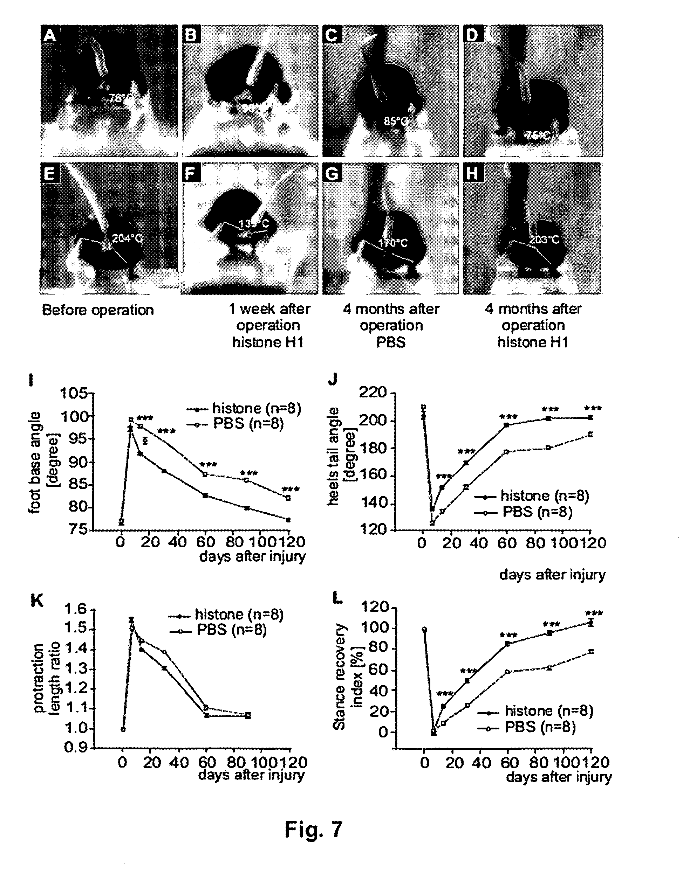

**Fig. 7****:** *Analysis of motor function after femoral nerve injury and application of histone H1.* **(A-H)** Foot-base angle (FBA) **(A-D)** and heels-tail angle (HTA) **(E-H)** were determined by analyzing single video frames from recordings of beam walking of mice prior to injury **(A, E)**, one week (B, F) and four months **(C, D, G, H)** after injury and application of histone H1 **(B, D, F, H)** or vehicle solution PBS **(C, G)** to the lesion site. **(I-L)** Time course of motor recovery after application of histone H1 or PBS are shown as mean values ±SEM of foot-base angle **(I)** and heels-tail angle **(J)**, protraction length ratio **(K)** and stance recovery index **(L)** at different time points after injury and application of histone H1 in PBS or PBS. Pre-injury values are plotted for day 0 and for other time points indicated in the graphs. Numbers of animals studied per group are indicated. Asterisks indicate significant differences (p<0.05, one-way ANOVA with Tukey's post-hoc test) between the histone H1-treated group and the PBS control groups at the given time points.

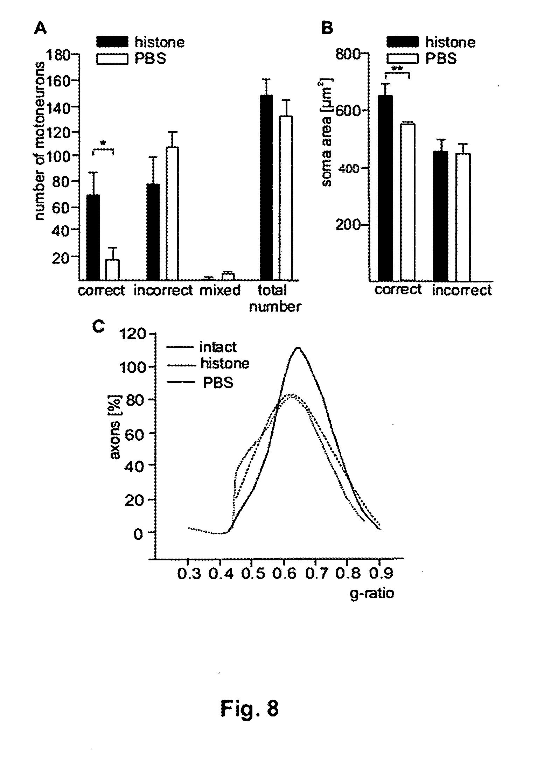

**Fig. 8****:** *Analysis of regrowth of motoneuron axons after femoral nerve injury.* Four months after injury and application of histone H1 or vehicle (PBS), animals were subjected to retrolabeling of motoneurons. **(A)** Mean numbers ±SEM of motoneurons labelled through the motor branch representing correctly projecting neurons (correct), through the sensory branch representing incorrectly projecting neurons (incorrect), through both branches (mixed) and the sum of labeled neurons (total number) are shown. (B) Morphometric analysis of soma size of regenerated motoneurons was performed. Mean values ±SEM of soma area of correctly and incorrectly projecting motoneurons after application of histone H1 or PBS are shown. **(A, B)** Significant differences between the groups of eight animals were found *(p*>0.05, one-way ANOVA with Tukey's *post hoc* test). **(C)** Analysis of myelinated nerve fibres in regenerated and intact nerves. Shown are normalized frequency distributions of g-ratios (axon/fiber diameter) in the motor nerve branch of non-injured mice (intact) and mice treated with histone H1 (histone) or vehicle (PBS) 4 month after injury.

### Examples

### Antibodies and reagents

Rabbit polyclonal antibody against human histone H1 (Santa Cruz Biotechnology, Heidelberg, Germany), mouse monoclonal antibody AE-4 against histone H1 (Millipore/Upstate, Schwalbach, Germany), horseradish peroxidase (HRP) conjugated NeutrAvidin (Thermo Scientific/Pierce, Bonn, Germany), HNK-1 antibody 412 (Kruse et al., 1984) and mouse antibody G3G4 against BrdU (Developmental Studies Hybridoma Bank, Iowa City, IA, USA) were used. Mouse monoclonal IgG2a antibody 735 against PSA (Frosch et al., 1985) and endoglycosidase N was a kind gift from Dr. Rita Gerardy-Schahn, (Zentrum Biochemie, Zelluläre Chemie, Medizinische Hochschule, Hannover, Germany). Secondary antibodies were purchased from Dianova (Dianova USA Inc.New york, USA). Histone H1 (#14-155, bovine histone H1.4) was purchased from Millipore/Upstate (Millipore) and glyceraldehyde-3-phosphate dehydrogenase (GAPDH), colominic acid, chondroitin sulfate A and C and heparin are from Sigma-Aldrich, Steinheim, Germany.

### Propagation of phage library

The "Griffin.1" phage scFv library (Griffiths et al., 1994) consisting of ∼10⁶ individual synthetic human scFv antibodies cloned into the phagemid pHEN2 was a kind gift from Dr. Greg Winter (Medical Research Council Center for Protein Engineering, Cambridge, England). The suppresser *E*. *coli* strain TG1 (K12, *supE, hsdD5*/ *F`, thi,* Δ*(lac⁻ pro) , traD36, proA+B+, lacl^{q}, lacZΔ M15)* was infected with the "Griffin.1" phage scFv library and grown in 2TY medium (16 g/L trypton, 10 g/L yeast extract, 5 g/L NaCl) supplemented with 100µg/ml ampicillin and 1% glucose. When reaching exponential growth (OD₆₀₀= 0.5) 2 x 10¹¹ M13K07 helper phages (GE Healthcare, Uppsala, Sweden) were applied to 25 ml culture (corresponding to 1 x 10¹⁰ bacteria). After incubation for 30 min at 37°C the infected bacteria were centrifuged for 10 min at 3300 g and the pellet was incubated in 2YT medium supplemented with 100 µg/ml ampicillin and 25 µg/ml kanamycin overnight at 30°C. The bacteria were pelleted at 10,800 g for 10 min at 4°C and 0.2 volumes of PEG/NaCl (20% PEG6000 in 2.5 M NaCl) was added to the resulting supernatant containing the phages. After incubation on ice for 1 h, phages were pelleted by centrifugation at 10,800 g for 30 min and the resulting pellet was resuspended in 40 ml H₂O and 8 ml PEG/NaCl and incubated for 20 min at 4 °C. After centrifugation at 10, 800 g for 30 min the phage pellet was resuspended in 5 ml PBS (8.1 mM Na₂HPO₄, 1.74 mM NaH₂PO₄, 0.15 M NaCl, pH 7.5).

### Selection of PSA binding scFv antibodies

PSA antibody 735 (100 µg/ml) dilute in 4 ml PBS was incubated in immunotubes (Maxisorb, Nunc, GmbH & Co. KG, Langenselbold,Germany) overnight at 4°C. After three washes with PBS, the tubes were blocked with 2% skim milk (Frema Reform, DE-VAU-GE, Lüneburg, Germany) in PBS (MPBS) for 2 h at room temperature. In parallel, an immunotube was blocked with 2% MPBS and incubated with 4 ml PBS containing ∼4 × 10¹³ phages, 2 mg mouse serum (Sigma-Aldrich) and 24 µg IgG2a (Cymbus Biotechnology, Hampshire, UK) for 1 h at room temperature. After incubation of the tube with PSA antibody, the tube was washed three times with PBS and incubated with the preincubated phage suspension at room temperature for 30 min on a head-over-top rotator and 1.5 h without agitation. After incubation, the tube was washed 20 times with PBS containing 0.1% Tween-20, followed by 20 washes with PBS. Bound phages were eluted with 1 ml 100 mM triethylamine and immediately neutralized with 0.5 ml 1 M Tris/HCI, pH 7.4. The eluted phages were used to infect exponentially growing *E. coli* TG1 cells, and infected bacteria were grown overnight at 30°C on TYE plates (15 g/L agar, 10 g/L yeast extract, 8 g/L NaCl) containing 100 µg/ml ampicillin and 1% glucose. After four rounds of selection, individual clones were analyzed by phage ELISA.

### Phage ELISA

Microtiter plates (Maxisorb, Nunc) were coated overnight at 4°C with 50 µl purified PSA antibody in PBS (100 µg/ml) per well. All following steps were done at room temperature. The plates were blocked with 2% MPBS. As negative controls 50 µl mouse serum (500 µg/ml) and IgG2a (25 µg/ml) were used. After blocking with 2% MPBS for 1 h at room temperature, plates were incubated with bacterial supernatants containing selected phages. After 6 washes with PBS, bound phages were detected using a horse radish peroxidase (HRP)-conjugated monoclonal antibody against M13 phage (GE Healthcare) and 2,2'-azino-bis(3-ethylbenzthiazoline-6-sulphonic acid) as substrate. Absorption at 405 nm was measured.

### DNA sequencing and truncation of the scFv linker region

The sequences of selected clones were determined with the primers LMB3 (5'-CAG GAA ACA GCT ATG AC-3') (SEQ ID NO:10) and fdSeq1 (5'-GAA TTT TCT GTA TGA GG-3' ) (SEQ ID NO:11) by the dideoxy chain terminating method using a model 377 DNA sequencer from Applied Biosystems (Foster City, CA, USA). For truncation of the linker region from 14 amino acids (SSGGGGSGGGGSGG) (SEQ ID NO:1) to 5 amino acids (SSGGG) (SEQ ID NO:2), pHEN2-derived plasmid DNA deriving from selected phages were subjected to PCR using the primers 5' -CAG CCG GCC ATG GCC CAG GTG- 3' (SEQ ID NO:3) and 5'GAG TCA CCG TGC ACT GCC TCC ACC ACT CGA GAC GGT GAC CAG GGT-3' (SEQ ID NO:4). The PCR product and the pHEN2 phagederived plasmids were cut with NcoI and ApaL1 and ligated.

### Production and purification of scFv antibodies

The nonsuppressor strain HB2151 (K12, *ara, thi*/ *F', proA+B+,* Δ *(lac⁻ pro), lacl^{q}, lacZ*Δ*M15)* was infected with selected phages to produce soluble scFv antibodies due to an amber stop codon in the pHEN2 sequence between the scFv gene and the M13 minor coat protein III gene. Bacteria transformed with selected phages were grown at 37°C in LB medium containing 0.1% glucose and 100 µg/ml ampicillin. At OD₆₀₀ 0.8-0.9, production of scFv antibodies was induced by addition of isopropyl-β-D-thiogalactopyranoside at a final concentration of 1 mM. After an overnight incubation at 30°C, culture supernatants were concentrated by ultrafiltration and applied to Ni-NTA agarose beads (Qiagen, Hilden, Germany) which were equilibrated with 50 mM phosphate buffer, pH 7.5, 300 mM NaCl, and 10 mM imidazole. After an overnight incubation at 4°C, the beads were centrifuged at 2500 g for 5 min and washed with 50 mM phosphate buffer, pH 7.5, 300 mM NaCl, and 20 mM imidazole. The scFv antibodies were eluted by incubating beads with 50 mM phosphate buffer, pH 7.5, 300 mM NaCl, and 250 mM imidazole for 30 min at 4°C and the eluted scFv antibodies were concentrated by centrifugation in Centricon-5 tubes (5 kD cut off; Millipore-Amicon) and diluted in PBS.

### ELISA using scFv antibodies

96-well microtiter plates (Nunc) were coated with 100 µl purified PSA antibody (100 µg/ml PBS) overnight at 4°C and blocked with 2% MPBS. Purified scFv antibodies supplemented with 2% skimmed milk were added to the plates and incubated for 1.5 h. For competition, colominic acid, chondroitin sulfate A and C or heparin were preincubated with the coated antibody before addition of scFv antibodies. After washing three times with 0.05% Tween 20 in PBS and three times with PBS, anti-myc tag antibody (Santa Cruz Biotechnology) was added and incubated for 1 h. The wells were washed again and incubated with HRP-conjugated secondary antibody for 1 h. Binding was detected using 2,2'-azino-bis(3-ethylbenzthiazoline-6-sulphonic acid) as substrate. All steps were carried out at room temperature.

### Surface plasmon resonance

The PSA antibody 735 was immobilized on a CM5 sensor chip (Biacore, Uppsala, Sweden). Purified scFv antibodies in PBS were injected and sensorgrams were recorded using a Biacore 3000 instrument. For competition, 5 µM of scFv antibody were mixed with the competitor before injection.

### Preparation of crude membrane fraction, synaptosomes and membrane-associated proteins

Postnatal (7 days old) or and adult (12-16 weeks old) C57BL/6J mice were decapitated; brains were immediately transferred into a homogenizer (Teflon pestle, 0.1µm; Wheaton, Millville, USA) and homogenized in 3 ml of homogenization buffer (0.32 M sucrose, 1 mM CaCl₂, 1 mM MgCl₂, 5 mM Tris/HC1, pH 7.5, and protease inhibitor (Roche, Mannheim, Germany) and applying 10-12 up-and-down strokes. This step and all following steps were carried out on ice or at 4°C. The homogenate was centrifuged for 10 min at 17,000 g. The pellet was re-suspended in homogenization buffer, applied to a 1.2 M, 1.0 M and 0.8 M sucrose step gradient, and centrifuged for 2 h at 100,000 g. The material from the 1.0/1.2 M sucrose interface was collected, diluted and pelleted by centrifugation for 20 min at 17,000 g. The pellet enriched in synaptosomal membranes was re-suspended in PBS containing 1% Triton X-100 and incubated for 30 min. This fraction was taken as a detergent-solubilized synaptosomal membrane subfraction.

For the preparation of membrane-associated proteins, brains were homogenized in 4 ml TNE buffer (25 mM Tris-HC1, pH 7.5, 150 mM NaCl, 5 mM EDTA) using a homogenizer. The homogenates were centrifuged at 1000 g for 10 min and the resulting supernatant was centrifuged at 100,000 g for 30 min. The pellet was resuspended in 2 ml TNE and adjusted to 150 mM NaHCO₃, pH 11.5. After incubation for 30 min the sample was subjected to centrifugation at 100,000g for 30 min. The resulting supernatant was taken as a subfraction enriched in membrane-associated protein.

### Affinity chromatography with scFv antibodies

The fraction containing membrane-associated proteins was first run over a Sepharose 4B column (GE Healthcare) and then run over a column with 3.2 mg purified scFv antibodies immobilized on 2 ml CNBr-activated Sepharose 4B (GE Healthcare) overnight at 4°C (0.2 ml/min). After washing the column with 15 ml PBS (0.3 ml/min) bound proteins were eluted with 200 mM glycine/ 500 mM NaCl, pH 2.7 (0.3 ml/min). The collected elution fractions were neutralized immediately with 1 M Tris/HC1, pH 8.8. After ultrafiltration using Centricon-5 tubes (Millipore-Amicon) the proteins were analyzed by SDS-PAGE, silver staining and mass spectrometry.

### Silver staining

Gels were incubated in methanol/acetic acid/water (50:5:45) for 30 min, washed two times with water, incubated in water for 1 h and exposed for 1 min to 0.02% sodium thiosulfate. After washing two times with water, the gels were incubated with pre-cooled 0.1% silver nitrate for 30 min at 4°C and washed two times with water. Color development was started by addition of 2% sodium carbonate/0.04% formaldehyde and stopped by addition of 2.3 M citric acid.

### Preparation of post-synaptic densities

Postsynaptic densities (PSDs) were prepared according to a slightly modified protocol previously described (Cohen et al., 1977). All steps were carried out at 4°C or on ice. Brains were homogenized in 3 ml homogenization buffer per brain. The homogenates were centrifuged at 1000 g for 10 min. The supernatant (S1) was saved and the pellet was again homogenized with three strokes. After centrifugation at 1000 g for 10 minutes, the supernatant was pooled with S1 and spun at 1000 g. The resulting supernatant was then centrifuged at 17,000 g and the pellets were re-suspended in homogenization buffer and centrifuged at 17,000 g for 10 min. The pellets were re-suspended in solution A (0.32 M sucrose and 1mM NaHCO₃) and applied to a 0.85 M, 1.0 M, and 1.2 M sucrose gradient. The gradients were run for 2 h at 100,000 g. The band between 1.0 and 1.2 M sucrose containing synaptosomes was then re-suspended in 4 volumes of solution A and centrifuged at 48,200 g for 20 min. The supernatant was re-suspended in solution B (0.5% Triton X-100 in 0.16 M sucrose, 6 mM Tris/HC1, pH 8.1) and centrifuged at 48,200 g for 20 min. The pellet was re-suspended in 2.5 ml of solution A, and 2 ml of this material was layered onto gradients composed of 3 ml of 2.0 M sucrose, 3.5 ml of 1.5 M sucrose and 3.5 ml of 1.0 M sucrose all containing 1 mM NaHCO₃. The gradients were spun for 2 h at 275,000 g. The PSDs banded at the interphase of 1.5 M and 2.0 M sucrose.

### SDS-PAGE and Western blot analysis

Proteins were separated by sodium dodecyl sulfate polyacrylamide gel electrophoresis (SDS-PAGE) using acrylamide slab gels and transferred to nitrocellulose membranes (Protran, Schleicher & Schuell, Dassel, Germany). Membranes were blocked in 5% milk powder in PBS for 1 h at room temperature and incubated with primary antibodies in blocking buffer overnight at 4°C. The appropriate HRP-conjugated secondary antibodies (Dianova) were applied after six washing steps with PBS containing 0.1% Tween-20. After incubation for 1 h at room temperature with secondary antibodies, membranes were washed again with PBS containing 0.1% Tween-20 for six times and blots were developed using enhanced chemiluminescence (Thermo Scientific).

### ELISA

384-well flat bottom micro titer plates (Corning, Lowell, MA, USA) were coated in triplicates with 25 µl of histone H1 and GAPDH (10 µg/ml in PBS) or PBS alone as control at 4°C overnight. After blocking for 1 h at room temperature with PBS containing 1% BSA, the wells were washed 3 times with PBS containing 0.05% Tween-20 (PBST). Wells were incubated with increasing concentration of soluble colominic acid for 1 h at room temperature. After wells were washed 5 times with PBST and 2 times with PBS, 25 µl of the PSA antibody 735 (5 µg/ml) were added to each well. After 1 h incubation of wells at room temperature, the plates were washed 6 times with PBST and 2 times with PBS. 25 µl of HRP-conjugated anti-mouse IgG were added to each well and incubated at room temperature for 1 h and then washed again. Antibody binding was visualized by incubating wells with 50 µl of 0.5 mg/ml o-phenylenediamine dihydrochloride (Thermo Scientific) for 2 to 5 min. The reaction was stopped by addition of 50 µl 2.5 M H₂SO₄ and quantified using an ELISA reader at 490 nm and the software KCjunior (Bio-TEK, Winooski, VT, USA).

### Neurite outgrowth assays for cerebellar neurons

Primary cultures of cerebellar granule cells were seeded at a density of 2 10⁵ cells/ml into 48-well plates (Nunc) and pre-treated with 0.01% poly-L-lysine (PLL) overnight at 4°C, washed twice with water, and air-dried. Histone H1 and laminin were substrate-coated, i.e., coated onto the surfaces overnight at a concentration of 10 µg/ml onto the dried surfaces at 4°C. The plates were washed two times with PBS, and neurons were plated at a density of 10⁵ cells/well in 300 µl of chemically defined serum-free culture medium. For antibody blocking experiments, rabbit polyclonal antibodies against histone H1 or PSA antibody 735 were added to the wells at a concentration of 10 µg/ml or 40 µg/ml one hour after cells had been seeded. At the same time, cells were treated with soluble histone H1 and/or soluble colominic acid (10 µg/ml). Endoglycosidase N was added to the neurons at a concentration of 4.8 µg/ml. After 24 h, cells were fixed by the gentle addition of 25% glutaraldehyde to a final concentration of 2.5% (Agar Scientific, Stansted, UK). After fixation, cultures were stained with 1% methylen blue/toluidine blue (Sigma-Aldrich) in 1% borax. Morphological parameters were quantified with the Axiovert microscope and the AxioVision image analysis system 4.6 (Carl Zeiss Micro Imaging, Göttingen, Germany). For morphometric analysis, only cells without contact with other cells were evaluated. Neurites were defined as those processes with a length of at least one cell body diameter. To determine the total neurite length per cell, 50 cells in each of two wells were analyzed per experiment.

### Schwann cell culture and analysis of process elongation

Schwann cells were isolated from dorsal root ganglia (DRG) of 7-day-old C57BL/6J mice. Tissues were removed, washed once with ice-cooled Ham's F-12 (PAA Laboratories, Cölbe, Germany), and then incubated with 0.25% trypsin and 0.1% collagenase (Sigma-Aldrich) at 37°C for 30 min. After enzymatic digestion, tissues were washed twice with ice-cooled Ham's F-12 medium and then suspended in 1 ml Ham's F-12 medium containing 0.01% DNase (Sigma-Aldrich). Mechanical digestion was performed using fire-polished Pasteur pipettes, and cells were suspended in 5 ml Ham's F-12 medium, added on top of 5 ml of a 4% bovine serum albumin (BSA, fraction V, PAA Laboratories) cushion and centrifuged for 10 min at 4°C and 500 g. Finally, Schwann cells were suspended in fresh pre-warmed (37°C) medium and seeded on 48-well plates (Nunc), that were pre-treated with 0.01% poly-L-lysine (PLL) and coated with laminin (10 µg/ml, Sigma-Aldrich) or purified histone H1. The medium used for Schwann cell culture contained DMEM high glucose/Ham's F-12 (1:1) (PAA Laboratories), 60 ng/ml progesterone (Sigma-Aldrich), 16 µg/ml putrescine (Sigma-Aldrich), 5 µg/ml insulin (Sigma-Aldrich), 0.4 µg/ml L-thyroxine (Sigma-Aldrich), 160 ng/ml sodium selenite (Sigma-Aldrich) , 10 ng/ml triiodothyronine (Sigma-Aldrich), 38 ng/ml dexamethasone (Sigma-Aldrich), 100 U/ml penicillin (PAA Laboratories), 100 µg/ml streptomycin (PAA Laboratories ) and 2 mM L-glutamine (PAA Laboratories ). Schwann cells were seeded at a density of 50,000 cells/ml. Cells were treated with soluble histone H1 and/or soluble colominic acid (10 µg/ml) 1 h after cells had been seeded. After incubation for 24 h at 37°C, cells were fixed with 2.5% glutaraldehyde and stained with 1% methylen blue/toluidine blue in 1% borax. The length of processes was measured by using the Axiovert microscope and the AxioVision image analysis system 4.6. For morphometric analysis, only cells without contact with other cells were evaluated. Processes were defined as those processes with a length of at least one cell body diameter. To determine the total neurite length per cell, 50 cells in each of two wells were analyzed per experiment.

### Schwann cell proliferation

Schwann cells were seeded at a density of 250,000 cells/ml onto 12-well plates (Nunc), containing 12 mm glass coverslips pre-treated with 0.01% poly-L-lysine (PLL) and coated with laminin and purified histone H1 at a concentration of 10 µg/ml. The cells were cultured in the presence of neuroregulin (12 ng/ml, ImmunoTools, Friesoythe, Germany). 20 µM BrdU (Sigma-Aldrich ) was added to the culture 4 h after seeding. One hour after BrdU treatment, cells were treated with soluble histone H1 and/or soluble colominic acid at a concentration of 10 µg/ml and then cultured for 48 h. Cells were washed shortly with PBS and fixed with 4% formaldehyde in 0.1 M phosphate buffer, pH 7.3. After incubation for 30 min with 2 N HCl at 37°C, cells were washed, blocked with goat serum and incubated overnight with mouse primary antibodies G3G4 against BrdU (1:100 dilution). Appropriate secondary antibodies coupled to fluorescent dye Cy3 were applied to the cells in the dilution 1:200 for 1 h at room temperature in the dark. The coverslips were finally washed and mounted with Fluoromount-G (Southern Biotech, Birmingham, UK). To estimate numbers of proliferating cells, 10 photographs per treatment were taken from different areas of the coverslip using an Axiophot 2 microscope (Carl Zeiss MicroImaging, Inc.Thornwood, NY 10594, USA) and a 20x objective. Each area was photographed using phase contrast and epifluorescence. The two digital images were then overlaid using the Image J software (http://rsbweb.nih.gov/ij/download.html) and Image Tool 2.0 software (University of Texas, San Antonia, TX, USA, http://ddsdx.uthscsa.edu/dig/) was used to count BrdU-positive Schwann cells and total number of Schwann cells. Schwann cells in culture have a long spindle-shaped cell body and two processes in opposite directions, which makes them easily distinguishable from other contaminating cells (DRG neurons and fibroblasts). Approximately 1,000 cells were counted for each experimental value.

### Immunostaining of live Schwann cells and cerebellar neurons

2 x 10⁵ Schwann cells or cerebellar neurons were seeded on 12 mm glass coverslips pre-treated with 0.01% PLL. 24 h after seeding, cells were washed three times with the corresponding culture medium containing 1% BSA. Cells were incubated on ice for 20 min with the primary antibodies against PSA and histone H1. Cells were then washed gently with serum-containing medium and appropriate secondary antibodies coupled to fluorescent dyes Cy3 and Cy 2 were applied to the cells for 20 min at room temperature in the dark. After washing with serum-free culture medium, cells were fixed with 4% formaldehyde for 15 min at room temperature, washed again three times with PBS. Cells were stained with DAPI for 2 min and finally washed three times with PBS and then mounted with Fluoromount-G. Images were taken with an Olympus Fluoview 1000 microscope (Olympus, Hamburg, Germany).

### Preparation and passage of neural precursor cells

Mouse embryos (E13.5) were decapitated, mid-dissection-embryo brain skin was exposed and skull peeled away. Developing striatum were dissected and transferred to serum-free media and then mechanically dissociated by pipetting up and down with three fire-polished Pasteur pipettes with sequentially smaller diameters. After dissociation, the neurospheres were centrifuged at 200g for 1 min at room temperature, re-suspended with 0.5 ml of accutase (Invitrogen GmbH, Karlsruhe ,Germany) and incubated at 37°C for 10 min. 0.5 ml of neural stem cell medium was applied to the cells and pipetted up and down for 15 to 20 times. After centrifugation at 200g for 5 minutes at room temperature, cells were re-suspended in 5 ml of neural stem cell medium and diluted to 10⁵ cells/ml of neural stem cell medium.

### Immunostaining of neurospheres

Neurospheres were allowed to settle for 10 minutes after culture in neurosphere-formation medium (DMEM/F12 (1:1) without L-glutamine, 1x B27 supplement (Invitrogen GmbH,), 0.02% sodium bicarbonate (GIBCO, Invitrogen GmbH), 0.5 M HEPES (Sigma-Aldrich), 16.6 mM glucose (Sigma-Aldrich), 100 mM glutamine (Invitrogen GmbH), 20 ng/ml EGF, 20 ng/ml FGF-2 for 4-5 days. After washing three times in pre-cooled PBS, neurospheres were fixed with 4% formaldehyde in filtered 0.4 M cacodylate buffer, pH 7.3, at 4°C for 1 h and dehydrated with 30% sucrose in cacodylate buffer at 4°C for 4 h. The neurospheres were embedded in tissue-tag, frozen at -30°C and sectioned at 14 µm on a cryostat (Leica CM 3050, Leica instruments GmbH, Nuβloch, Germany). Sections were blocked with 10% goat serum and 0.1% Triton X-100 at room temperature for 1 h before being incubated with primary antibodies against PSA and histone H1 at 4°C overnight. After washing three times with PBS, appropriate secondary antibodies coupled to the fluorescent dyes Cy3 and Cy 2 were applied to the cells for 1 h at room temperature in the dark. Washed three times with PBS, the sections were labeled with bisbenzimide (Sigma-Aldrich) for nuclei staining and mounted with Fluoromount-G after additional washes in PBS. Images were taken with an Olympus Fluoview 1000 microscope (Olympus).

### Neurosphere migration assay

After 10 to 15 days of culture *in vitro* in neurosphere-formation medium, the cell migration assay was performed according to Decker et al. (2000). Briefly, the non-adherent neurospheres were seeded in 10 µl droplets on glass coverslips pre-treated with 0.02% PLL followed by laminin and histone H1 as substrates. Neurospheres were incubated at 37°C for 20 min. The medium was adjusted to 1 ml per well with neurosphere-formation medium (without FGF-2 and EGF) containing soluble colominic acid and/or histone H1. After 24 h, migration was assessed by measurement of the distance from the edge of the neurosphere to all cells having migrated out of the neurosphere using an Axiovert microscope and the AxioVision image analysis system 4.6 (Carl Zeiss Vision GmbH).

### Animals and surgical procedures

Femoral injury and analysis of regeneration were performed according to simova et al., (2006). Three-month-old female C57BL/6J mice were obtained from the central animal facility of the Universitätsklinikum Hamburg-Eppendorf. All experiments were conducted in accordance with the German and European Community laws on protection of experimental animals. The procedures used were approved by the responsible committee of The State of Hamburg. For surgery, the animals were anaesthetized by intraperitoneal injections of 0.4 mg kg⁻¹ fentanyl (Fentanyl-Janssen, Janssen-Cilag, Neuss, Germany), 10 mg kg⁻¹ droperidol (Dehydro-benzperidol, OTL Pharma, Paris, France) and 5 mg kg⁻¹ diazepam (Ratiopharm, Ulm, Germany). The right femoral nerve was exposed and nerve transection performed at a distance of approximately 3 mm proximal to the bifurcation of the nerve into motor and sensory branches. A polyethylene tubing (3 mm length, 0.58 mm inner diameter; Becton Dickinson, Heidelberg, Germany) was placed between the two nerve stumps and filled with PBS containing scaffold peptide that forms a gel matrix support (0.5% PuraMatrix Peptide Hydrogel, 3D, BD Biosciences, San Jose, California,USA), or PBS/scaffold peptide supplemented with histone H1 (100 µg/ml). The cut ends of the nerve were inserted into the tube and fixed with single epineural 11-0 nylon stitches (Ethicon, Norderstedt, Germany) so that a 2 mm gap was present between the proximal and distal nerve stumps. Finally, the skin wound was closed with 6-0 sutures (Ethicon).

### Analysis of motor function

Functional recovery was assessed by single-frame motion analysis (Irintchev et al., 2005; Ahlborn et al., 2007) to evaluate quadriceps muscle function during ground locomotion. Prior to operation, mice were trained to perform a beam-walking test in which the animal walks unforced from one end of a horizontal beam (length 1000 mm, width 38 mm) towards its home cage located at the other end of the beam. A rear view of one walking trial per animal was captured prior to the operation with a high-speed camera (A602fc, Basler, Ahrensburg, Germany) at 100 frames per second and stored on a personal computer in Audio Video Interleaved (AVI) format. The recordings were repeated 1, 2, 4, 8, 12 and 16 weeks after nerve transection. The video sequences were examined using SIMI-Motion 7.0 software (SIMI Reality Motion Systems, Unterschleissheim, Germany). Selected frames in which the animals were seen in defined phases of the step cycle were used for measurements of two parameters as described previously (Irintchev et al., 2005): the foot-base angle (FBA) and the heels-tail angle (HTA). The analysis was performed with UTHSCSA Image Tool 2.0 software (University of Texas, San Antonia, TX, USA, http://ddsdx.uthscsa.edu/dig/). Both parameters are directly related to the ability of the quadriceps muscle innervated by the motor branch of the femoral nerve to keep the knee joint extended during contralateral swing phases. As a relative measure of functional recovery at different time-point after nerve injury, we calculated the stance recovery index, which is a mean of the recovery index (RI) for the HTA and the FBA. The index for each angle is calculated in percent as: RI= [ (*X*ᵣₑᵢₙₙ―*X*_{den}) / (*X*ₚᵣₑ―*X*_{den}) ] 100, where *X*ₚᵣₑ, *X*_{den} and *X*ᵣₑᵢₙₙ are values prior to operation, during the state of denervation (7 days after injury), and at any given time-point during reinnervation, respectively. A third parameter, the limb protraction length ratio (PLR), was evaluated from video recordings of voluntary pursuit movements of the mice. The mouse, when held by its tail and allowed to grasp a pencil with its fore paws, tries to catch the object with its hind paws and extends simultaneously both hind limbs. In intact animals the relative length of the two extremities, as estimated by lines connecting the most distal mid-point of the extremity with the anus, is approximately equal and the PLR (ratio of the right to left limb length) is close to 1. After unilateral injury, the denervated limb cannot extend maximally and the PLR increases significantly above 1.

### Retrograde labelling and evaluation of motoneuron number and soma size

Following the last video recording, mice were re-operated for retrograde labelling. Under fentanyl/droperidol/diazepam anaesthesia, the two nerve branches were transected ∼5 mm distal to the bifurcation, and two fluorescent dyes, Fast Blue (EMS-Chemie, Großumstadt, Germany) and Fluoro-Gold (Fluorochrome, Denver, CO, USA), were applied to the motor and sensory branches, respectively. One week later, the animals were perfused with 4% formaldehyde (Sigma-Aldrich) in 0.1 M sodium cacodylate buffer, pH 7.3, and spinal cords and femoral nerves were dissected for morphological analyses. After overnight fixation, the lumbar part of the spinal cord was cut transversely (serial sections of 50 µm thickness) on a Leica vibratome VT1000S (Leica CM 3050,Leica instruments GmbH). The sections were examined under a fluorescence microscope (Axiophot 2, Zeiss) with appropriate filter sets. All cell profiles labeled with one of the dyes or with both tracers are distributed within a stack of 35-45 serial cross-sections. Each section, containing typically 2-5 labeled cell profiles, was examined using a 40x objective by focusing through the section thickness starting from the top surface. All profiles except those visible at the top surfaces of sections were counted (Simova et al., 2006). The application of this simple stereological principle prevents double counting of labelled cells and allows an unbiased evaluation of cell numbers, which does not rely on assumptions or requires corrections. The same sections were used for measurements of soma size using Neurolucida software-controlled computer system (MicroBrightField Europe, Magdeburg, Germany).

### Analysis of the degree of myelination

After fixation with 4% formaldehyde, femoral nerves were post-fixed in 1% osmium tetroxide in 0.1 M sodium cacodylate buffer, pH 7.3, for 1 h at room temperature, dehydrated and embedded in resin according to standard protocols. Transverse 1 µm-thick sections from the motor and sensory nerve branches were cut (Ultra-microtome, (Leica CM 3050,Leica instruments GmbH) at a distance of ∼3 mm distal to the bifurcation and stained with 1% toluidine blue/1% borax in distilled water. Axonal and nerve fibre diameters were measured in a random sample from each section using a 100x oil objective and a grid with a line spacing of 30 µm projected into the visual field of an Axioskop microscope (Carl Zeiss Vision GmbH, Munich, Germany) equipped with a motorized stage and Neurolucida software (MicroBrightField Europe). Selection of the reference point (zero coordinates) of the grid was random. For all myelinated axons crossed by or attaching the vertical grid lines through the sections, mean orthogonal diameters of the axon (inside the myelin sheath) and of the nerve fiber (including the myelin sheath) were measured. The mean orthogonal diameter is calculated as a mean of the line connecting the two most distal points of the profile (longest axis) and the line passing through the middle of the longest axis at right angle. The degree of myelination was estimated by the ratio axon to fiber diameter (g-ratio).

### RESULTS

### Identification of an anti-idiotypic scFv antibody mimicking polysialic acid

To identify novel PSA binding partners, a PSA-mimicking anti-idiotypic scFv antibody for immunoaffinity chromatography was generated. First, scFv antibodies that bind to a PSA antibody had to be isolated. Therefore, a phage scFv library was screened for binding to immobilized PSA antibody. After four rounds of selection, ten clones were found to bind to PSA antibody. Four of these clones expressed the sequences LNDGPVTSA (SEQ ID NO:5), five clones expressed the sequence GLPIDS (SEQ ID NO:6) and one clone expressed SPFES (SEQ ID NO:7) in the CDR3 region of the V_{H} region of the scFv antibody. All clones consisted of a V_{H} and V_{L} chain deriving from the VH1 (DP-3) and VL1 (1-d) antibody gene family.

Next, it was checked by ELISA and surface plasmon resonance whether the binding of scFv antibodies deriving from the selected phages is PSA-specific. Since the yield of scFv antibody from the clone with the sequence SPFES (SEQ ID NO:7) in the CDR3 region was extremely low, this clone was not further analyzed. ELISA of the scFv antibody deriving from one of the five clones showing the sequence GLPIDS (SEQ ID NO:6) revealed that the binding of the scFv antibodies to substrate coated PSA antibody was inhibited in a concentration-dependent manner by the bacterial homolog of PSA, colominic acid, while other negatively charged polymers such as chondroitin sulfate A or C or heparin showed no inhibition of the binding (**Fig. 1A**)**.** In addition, the scFv antibody did not bind to HNK-1 antibody, which was used as a control antibody (data not shown). Surface plasmon resonance showed that the scFv antibody bound in a concentration-dependent manner to the immobilized PSA antibody (**Fig. 1B**) and that this binding was inhibited by increasing concentrations of colominic acid (**Fig. 1C**). The scFv antibody deriving from one of the four clones having the sequence LNDGPVTSA (SEQ ID NO:5) showed identical results (data not shown). These results indicate that the selected scFv antibodies showing the consensus motif P [VI] [TD] S mimic PSA.

To identify binding partners, ELISA was performed using purified scFv antibodies and different subfractions from mouse brain as substrate coats. No significant binding of scFv antibodies was observed (data not shown). The lack of binding could have been due to a low affinity of the scFv antibodies. This possibility was tested by increasing the avidity and thus the functional affinity of the scFv antibodies. Since truncation of the linker region between the V_{H} and V_{L} domains of the scFv antibody results in enhanced dimerization and/or oligomerization of scFv monomers and thus in an increased avidity (Holliger et al., 1993), the linker region of the selected phages was truncated (**Fig. 2A**). FPLC analysis of the scFv antibody with the sequence GLPIDS (SEQ ID NO:6) in the CDR3 region without and with linker truncation showed that the scFv antibody without truncation existed predominantly in a monomeric form with low amounts of the dimeric form, while the scFv antibody with the linker truncation existed only in a dimeric form (**Fig. 2B**)**.** The scFv with the sequence LNDGPVTSA (SEQ ID NO:5) in the CDR3 region also existed only in a dimeric form after linker truncation. The scFv antibodies with truncated linkers were analyzed by ELISA and surface plasmon resonance. In ELISA, the scFv antibody having the sequence GLPIDS (SEQ ID NO:6) in the CDR3 region of V_{H} showed a concentration-dependent and saturable binding to substrate coated PSA antibody (**Fig. 2C**). This binding was inhibited by colominic acid in a concentration-dependent manner, while neither the negatively charged polymers heparin, chondroitin sulfate A nor chondroitin sulfate C showed any inhibition (**Fig. 2D**). In surface plasmon resonance experiments the binding of scFv antibody to immobilized PSA antibody was inhibited by colominic acid in a concentration-dependent manner (**Fig. 2E**). Experiments with the scFv antibody with the sequence LNDGPVTSA (SEQ ID NO:5) showed identical results (data not shown). The k_{D} value for the binding of the scFv antibodies to the PSA antibody was estimated to be in the µM range and thus is similar to that determined for the binding of colominic acid to the PSA antibody (Evans et al., 1995). The scFv antibodies were applied to substrate coated brain subfractions. The scFv antibody with the sequence GLPIDS (SEQ ID NO:6) in the CDR3 region showed a strong binding to soluble proteins isolated from brain of postnatal and adult mice and a notable binding to detergent-solubilized membrane proteins, while the scFv antibody with the sequence LNDGPVTSA (SEQ ID NO:5) showed weak binding to soluble brain proteins and did not show binding to membrane proteins (**Fig. 3A**).

### Identification of histone H1 as synaptic membrane-associated PSA binding partner using a PSA-mimicking scFv antibody

Since the purified scFv antibody with the sequence GLPIDS (SEQ ID NO:6) in the CDR3 region showed binding to brain proteins, it was used for affinity chromatography using different brain subfractions. A ∼30 kDa protein band was detected by silver staining upon separating an eluate obtained after application of a subfraction which was enriched in membrane-associated proteins that were released from the membranes by alkaline treatment (**Fig. 3B**)**.** The ∼30 kDa band was analyzed by mass spectrometry. After tryptic digestion of the band two of the detected peptides could be assigned to histone H1 by ESI-MS/MS mass spectrometry. The MS/MS spectrums of a 1228.6936 Da and a 923.4724 Da precursor mass (detected as doubly charged ion at m/z=614.8 and 554.2) matched the tryptic peptide TSGPPVSELITK (SEQ ID NO:8) and ALAAAGYDVEK (SEQ ID NO:9) of histone H1. This result suggested that histone H1 is a binding partner of PSA that is tightly associated with brain membranes.

To verify the direct interaction between histone H1 and PSA, ELISA with purified colominic acid which is a bacterial derived PSA homologue was performed using purified histone H1 from calf thymus as substrate coat and GAPDH a negative control substrate coat. PSA directly binds to histone H1, but not GAPDH, in a concentration dependent manner (**Fig. 3C**), indicating that histone H1 and PSA interact directly.

To further confirm that histone H1 is tightly associated with the plasma membrane, the distribution of histone H1 in brain membrane subfractions was analyzed upon detergent treatment. Western blot analysis using a histone H1 specific antibody revealed that histone H1 was detected as ∼30 kDa protein only in the Triton X-100 insoluble subfraction enriched in synaptic membranes or in postsynaptic densities but not in the detergent soluble subfraction enriched in synaptic membranes (**Fig. 3D**). This result shows that histone H1 is associated with synaptic membranes and that it is tightly associated with detergent insoluble membrane microdomains like PSDs.

Since PSA is located extracellularly, we investigated whether its binding partner histone H1 is also present extracellularly. To test this, cell surface biotinylation using primary cerebellar neurons was performed and histone H1 was detectable as a biotinylated extracellular cell surface protein with an apparent molecular weight of ∼60 kDa, while it was detectable as a ∼30 kDa protein in the cell lysate (**Fig. 3E**). This result suggests that extracellular histone H1 exists in a stable dimeric form.

To further corroborate the notion that histone H1 interacts with PSA extracellularly, it was tested by live immunocytochemical staining whether histone H1 and PSA co-localize at the surface of cultured cerebellar neurons or Schwann cells. Confocal microscopic analysis using a polyclonal histone H1 antibody and a monoclonal PSA antibody showed histone H1 and PSA immunoreactivity both at the surface of cell bodies and along neurites of neurons (**Fig. 4A**) and processes of Schwann cells (**Fig. 4B**) and superimposed images demonstrated that a significant portion of histone H1 co-localizes with PSA (**Fig. 4A****, B**). The co-localization of histone H1 and PSA at the cell surface of live neural cells suggests that both molecules associate also *in vivo.*

### Histone H1 alters PSA induced neurite outgrowth of cerebellar neurons

Next, the functional role of extracellular histone H1 in neural cells was analyzed and it was investigated whether the interaction between PSA and histone H1 has functional consequences. First, we tested whether histone H1 influences neurite outgrowth (**Fig. 5A**)**.** Cerebellar neurons were grown on substrate-coated PLL or PLL with histone H1. In parallel, neurons were grown on PLL in the presence of soluble histone H1 and/or PSA. Neurite outgrowth on substrate-coated histone H1 was enhanced by approximately three-fold when compared to that seen on PLL substrate, and it reached values obtained for the positive control substrate laminin (Fig. 5A). Similarly, neurite outgrowth on PLL was increased approximately three-fold when soluble PSA was present, while a two-fold increase in neurite outgrowth was observed in the presence of soluble histone H1 (**Fig. 5A**)**,** indicating that soluble PSA and histone H1 individually trigger neurite outgrowth. However, in the concomitant presence of soluble histone H1 and PSA, neurite outgrowth was only slightly affected (**Fig. 5A**), indicating that soluble histone H1 and PSA interacted and were no longer able to trigger neurite outgrowth. To check whether endogenous extracellular histone H1 is involved in PSA-mediated neurite outgrowth, neurite outgrowth was performed in the absence or presence of soluble PSA and/or a polyclonal histone H1 antibody expected to bind to endogenous histone H1 and block its effect on neurite outgrowth. Addition of soluble histone H1 antibody significantly blocked the neurite outgrowth promoting effect of soluble PSA, while the antibody had no significant effect on neurite outgrowth in the absence of PSA (**Fig. 5A**)**.** These results indicate that interaction between histone H1 and PSA plays a crucial role in neurite outgrowth.

To demonstrate that histone H1 induced neurite outgrowth depends on the presence of PSA on the surface of cerebellar neurons, neurite lengths were measured in the presence of endoglycosidase N which degrades PSA or in the presence of the PSA-specific antibody 735. On PLL substrate-coat, neurite outgrowth in the presence of PSA antibody or endoglycosidase N was not altered in comparison to that observed in the absence of antibody and enzyme. However, the enhanced neurite outgrowth in the presence of histone H1 was reduced nearly to PLL level in the presence of either the PSA-specific antibody or the PSA-degrading enzyme. (**Fig. 5B**)**,** showing that that histone H1 induced neurite outgrowth is mediated by PSA present on the surface of neurons.

### Histone H1 and PSA influence process formation and proliferation of Schwann cells

The inventors showed that histone H1 and PSA co-localize on Schwann cell surface, and it has been shown that a PSA mimicking peptide and over-expression of PSA by Schwann cells improve myelination by Schwann cells in vivo (Mehanna et al., 2009a; Papastefanaki et al., 2007). These findings suggested that histone H1, like PSA, may affect Schwann cell process formation. To test this assumption, Schwann cells from dorsal root ganglia of early postnatal mice were cultured on substrate-coated PLL or histone H1 or in the presence of soluble histone H1 protein and/or soluble PSA, and the lengths of Schwann cell processes were measured. Morphometric analysis of Schwann cell process length revealed an increase of process length on substrate-coated histone H1 relative to PLL substrate and also in the presence of soluble PSA or soluble histone H1 (**Fig. 5C**). The concomitant presence of soluble PSA and histone H1 led to a neutralization of the promotion seen with the PSA and histone H1 when applied individually, indicating that the exogenously added histone H1 and PSA bound to each other and were no longer able to promote process extension.

The inventors also checked the effect of the histone H1 and PSA on Schwann cell proliferation by analysis of BrdU incorporation, and found that Schwann cell proliferation was enhanced by substrate-coated histone H1 and in the presence of soluble histone H1 and soluble PSA, when compared to proliferation in the absence of PSA and histone H1, respectively (**Fig. 5D**)**.** The mixture of soluble histone H1 and soluble PSA decreased the enhanced proliferation seen with histone H1 or PSA to levels observed on PLL substrate (**Fig. 5D**). These results show that PSA and histone H1 mediate proliferation and process formation of Schwann cells.

### Histone H1 and PSA alter migration of neural precursor cells

Previous studies have suggested that PSA is required for migration of neural precursors (Angata et al., 2007; Chazal et al., 2000; Hu, 2000; Hu et al., 1996; Patrzykat et al., 2001). Based on these studies, it was next tried to investigate whether histone H1 affects neural precursor cell migration. Beforehand, it was investigated by immunostaining whether neural precursor cells in neurospheres express histone H1 and PSA and observed that neural precursor cells not only express both histone H1 and PSA at their cell surface, but also that both protein partially co-localize (**Fig. 6A**). For analysis of migration, the neurospheres were maintained on substrate-coated histone H1, and in the presence of soluble histone H1 or soluble PSA. The number of cells migrating from the neurospheres and the distance between migrating cells and the edge of the neurospheres were determined and used as criteria for migration. On PLL, the number of cells migrated out of the neurosphere was low (**Fig. 6B**)**.** Migration of neural precursor cells was enhanced when either soluble PSA or soluble histone H1 was applied, while a mixture of PSA and histone H1 had only slightly affected migration (**Fig. 6B**)**.** As seen on the positive control substrate laminin, substrate-coated histone H1 remarkably enhanced migration (**Fig. 6B**). These findings indicate that both histone H1 and PSA are important for neural precursor cell migration *in vitro.*

### Histone H1 promotes functional recovery after femoral nerve injury in adult mice

Since PSA enhances regeneration after injury of the peripheral nervous system of adult mice (Mehanna et al., 2009a), and since the inventors have observed that histone H1 has beneficial effects on Schwann cells and neurons, the question was addressed whether histone H1 also enhances regeneration after femoral nerve injury. The function of the quadriceps muscle was evaluated during a four-month period after injury and after application of histone H1 or PBS. Femoral nerve injury in mice induces changes in gait which can be quantitatively evaluated by two parameters, the heels-tail angle (HTA) ](**Fig. 7A-D, I**) and the foot-base angle (FBA) (**Fig. 7E-H, J**) during beam walking (**Fig. 7A, H**). Changes in gait are caused by impaired extensor function of the quadriceps muscle leading to abnormal external rotation of the ankle and high heel position at defined gait cycle phases. These parameters were used to evaluate the effect of histone H1 on locomotor recovery. One week after injury, the degree of functional impairment, as evaluated by the increase of the foot-base angle (**Fig. 7B**) and decrease of the heels-tail angle (**Fig. 7F**) compared with the pre-operative values (**Fig. 7A,7E**) and found to be similar between groups of mice treated with histone H1 and PBS. When compared to the PBS control groups (**Fig. 7C, G- I, J**) better functional improvement was observed in histone H1 treated mice at 2 (**Fig. 7I,J**) , 3 (**Fig. 7I,J**) and 4 months (**Fig. 7D, H, I, J**) after injury. Analysis of the protraction length ratio, which is another parameter for functional recovery measuring the ability of a mouse to extend the hind limb to catch an object with its hind paws, revealed no statistically significant difference between the two groups. However, there was a tendency for better recovery of hind limb extension after application of histone H1 in comparison to PBS (**Fig. 7K**). Superior functional recovery after histone H1 treatment was apparent when the stance recovery index was calculated for both angles on an individual animal basis (**Fig. 7L**). Stance recovery index is a measure of the individual degree of recovery and reflects the degree of post-operative normalization of the quadriceps extensor function during ground locomotion. The recovery index of animals treated with histone H1 reached values at 3 and 4 months, which were not statistically different from the pre-operative values which are 100% 0.05, ANOVA and Tukey's post hoc test), indicating complete functional recovery. At the same time, recovery in the control group after 3 and 4 month was calculated as 62% and 76%, respectively. Thus, the overall results show that histone H1 treatment leads to a superior functional outcome.

### Histone H1 promotes survival, regrowth and reinnervation of motoneurons

To search for structural correlates of the superior functional recovery after application of histone H1, retrograde labeling of regenerated motoneurons was performed in animals after analyzing functional recovery. The number of motoneurons back-labeled through the motor (quadriceps) nerve branch which represent correctly projected regrown motoneurons significantly increased in animals to which histone H1 had been applied relative to PBS treated animals (**Fig. 8A**)**.** The number of back-labeled motoneurons from the sensory (saphenous) branch representing incorrectly projecting neurons decreased in animals treated with histone H1 in comparison to animals treated with PBS (**Fig. 8A**)**.** The small number of motoneurons back-labeled through both branches of the femoral nerve as well as the total number of labeled neurons were similar in histone H1 or vehicle treated mice (**Fig. 8A**)**.** Since the number of retrogradely labeled motoneurons reflects the extent of motoneuron survival (de la Cruz et al., 1994; Waters et al., 1998; Asahara et al., 1999), it can be concluded that treatment with histone H1 reduces motoneuron death, a characteristic feature of the femoral nerve injury paradigm in mice (Simova et al., 2006). On the other hand, histone H1 application improved motoneuron survival and also increased the number of regenerated motoneurons projecting to the appropriate motor nerve branch and thus increased preferential motor reinnervation, which is a characteristic feature of femoral nerve regeneration (Brushart, 1988; Al-Majed et al., 2000; Franz et al., 2005). In conclusion, the results show that better functional recovery in histone H1 treated mice is due to better motoneuron regeneration, since motoneuron loss was reduced and precision of motor reinnervation was improved as compared with control mice.

Since a significant correlations between recovery index and motoneuron size have been found 3 months after femoral nerve injury (Simova et al., 2006; Ahlborn et al., 2007), soma size of motoneurons regrowing their axons correctly was measured and a significant increase in soma size of correctly projecting motoneurons in histone H1 treated animals in comparison to PBS treated animals was found, while no difference in soma size of incorrectly projecting motoneurons was observed among the groups (**Fig. 8B**)**.** Therefore, better functional recovery in histone H1-treated mice can be attributed to larger motoneuron size, which is an indicator of a better functional state of regenerated motoneurons (Simova et al., 2006; Ahlborn et al., 2007).

Regenerated nerves were next analyzed morphometrically to assess the degree of myelination evaluated by the g-ratio (axon- to fiber-diameter ratio), and no significant between frequency distributions of the g-ratio for axons of intact and histone H1 or PBS treated injured nerves was found (**Fig. 8C**)**,** indicating that histone H1 does not effect myelination during regeneration.

## Claims

1. A pharmaceutical composition comprising a histone, a gene transfer vector for expression of a histone and/or a histone expressing cell for use in treatment or prevention of nerve injury.

2. The pharmaceutical composition of claim 1 comprising a histone.

3. The pharmaceutical composition of any of claims 1 or 2, wherein the histone is selected from the group consisting of histone H1, H2A, H2B, H3, H4, preferably, the histone is histone H1.

4. The pharmaceutical composition of any of the preceding claims, comprising one or more histones or a nucleosome.

5. The pharmaceutical composition of any of the preceding claims, wherein the histone is a human histone.

6. The pharmaceutical composition of any of the preceding claims, wherein the nerve injury is injury in the peripheral and/or central nervous system.

7. The pharmaceutical composition of any of the preceding claims, wherein the nerve injury comprises a lesion to the brain, spinal cord, sensory neurons and/or motoneurons.

8. The pharmaceutical composition of any of the preceding claims, wherein the nerve injury is inflicted by an operation, a disease and/or trauma.

9. The pharmaceutical composition of any of the preceding claims, wherein the nerve injury is acute injury.

10. The pharmaceutical composition of any of the preceding claims, wherein the nerve injury is chronic injury.

11. The pharmaceutical composition of any of the preceding claims, wherein the nerve injury is inflicted by a degenerative disease.

12. The pharmaceutical composition of any of the preceding claims, wherein the composition is formulated for administration to the peripheral or central nervous system by intraoperational administration, injection, intravenous injection or infusion into the diseased tissue.

13. The pharmaceutical composition of any of the preceding claims, wherein the composition is for inducing neurite outgrowth, for promoting process formation of Schwann cells, for promoting proliferation of Schwann cells, for enhancing migration and/or differentiation of neural progenitor cells, for promoting survival of neurons, for promoting regrowth of axons and dendrites, promoting functional recovery of injured neurons and/or fostering synapse formation and activity and synaptic plasticity.

14. The pharmaceutical composition of any of the preceding claims, wherein the composition is for enhancing learning and memory.

15. Use of a histone, a gene transfer vector for expression of a histone and/or a histone expressing cell for the preparation of the pharmaceutical composition of any of the preceding claims for the treatment and/or prevention of nerve injury.
